# EUROPEAN PATENT APPLICATION

(11) **EP 2 105 138 A2**
(43) Date of publication of application: **30.09.2009**
(21) Application number: 09009351.9
(22) Date of filing: 17.04.1997
(51) Int. Cl.: A61K 35/28, A61P 19/08

(54) **Regeneration and augmentation of bone using mesenchymal stem cells**

(30) Priority: 19.04.1996 US 16245 P; 28.10.1996 US 29838 P
(62) Divisional of application: 97920427.8
(71) Applicant: Osiris Therapeutics, Inc., Columbia, MD 21046 (US)
(72) Inventor: Kadiyala, Sudha, Baltimore, MD 21231 (US); Bruder, Scott P., Owing Mills MD 21117 (US); Muschler, George, F., Cleveland Heights, OH 44106 (US)
(74) Representative: Schnappauf, Georg

(57) **Abstract**

Disclosed are compositions and methods for augmenting bone formation by administering isolated human mesenchymal stem cells (hMSCs) with a ceramic material or matrix or by administering hMSCs; fresh, whole marrow; or combinations thereof in a resorbable biopolymer which supports their differentiation into the osteogenic lineage. Contemplated is the delivery of (i) isolated, culture-expanded, human mesenchymal stem cells; (ii) freshly aspirated bone marrow; or (iii) their combination in a carrier material or matrix to provide for improved bone fusion area and fusion mass, when compared to the matrix alone. The material or matrix can be a granular ceramic or three-dimensionally formed ceramic implant. The material or matrix can also be a resorbable biopolymer. The resorbable biopolymer is an absorbable gelatin, collagen or cellulose matrix, can be in the form of a powder or sponge, and is preferably a bovine skin-derived gelatin. The implants can be shaped as a cube, cylinder, block or an anatomical site. The compositions and methods can further include administering a bioactive factor such as a synthetic glucocorticoid, like dexamethasone, or a bone morphogenic protein, like BMP-2, BMP-3, BMP-4, BMP-6 and BMP-7.

## Description

This application is a continuation-in-part of U.S. provisional application serial no. 60/016,245, filed April 19, 1996 and U.S. provisional application serial no. 60/029,838 filed October 28, 1996.

Autologous, culture-expanded, bone marrow-derived MSCs have now been shown to regenerate clinically significant bone defects. Using techniques for isolating and cultivating human MSCs, it should be possible to implement therapeutic strategies based on the administration of a patient's own cells which have been harvested by a simple iliac crest aspiration. This method may provide an alternative to autogenous bone grafting, and will be particularly useful in clinical settings such as ageing and osteoporosis, where the number and/or function of endogenous MSCs have been reduced.

The repair of large segmental defects in diaphyseal bone is a significant problem faced by orthopaedic surgeons. Although such bone loss may occur as the result of acute injury, these massive defects commonly present secondary to congenital malformations, benign and malignant tumors, osseous infection, and fracture non-union. The use of fresh autologous bone graft material has been viewed as the historical standard of treatment but is associated with substantial morbidity including infection, malformation, pain, and loss of function (72,149). The complications resulting from graft harvest, combined with its limited supply, have inspired the development of alternative strategies for the repair of clinically significant bone defects. The primary approach to this problem has focused on the development of effective bone implant materials.

Three general classes of bone implants have emerged from these investigational efforts, and these classes may be categorized as osteoconductive, osteoinductive, or directly osteogenic. Allograft bone is probably the best known type of osteoconductive implant. Although widely used for many years, the risk of disease transmission, host rejection, and lack of osteoinduction compromise its desirability (76). Synthetic osteoconductive implants include titanium fibermetals and ceramics composed of hydroxyapatite and/or tricalcium phosphate. The favorably porous nature of these implants facilitate bony ingrowth, but their lack of osteoinductive potential limits their utility. A variety of osteoinductive compounds have also been studied, including demineralized bone matrix (34,45), which is known to contain bone morphogenic proteins (BMP). Since Urist's original discovery of BMP (138), others have characterized, cloned, expressed, and implanted purified or recombinant BMPs in orthotopic sites for the repair of large bone defects (44,126,146,147). The success of this approach has hinged on the presence of mesenchymal cells capable of responding to the inductive signal provided by the BMP (74,129). It is these mesenchymal progenitors which undergo osteogenic differentiation and are ultimately responsible for synthesizing new bone at the surgical site.

One alternative to the osteoinductive approach is the implantation of living cells which are directly osteogenic. Since bone marrow has been shown to contain a population of cells which possess osteogenic potential, some have devised experimental therapies based on the implantation of fresh autologous or syngeneic marrow at sites in need of skeletal repair (26,27,49,105,113,144,145). Though sound in principle, the practicality of obtaining enough bone marrow with the requisite number of osteoprogenitor cells is limiting.

### Summary of the Invention

The present invention provides compositions and methods for directing MSCs cultivated *in vitro* to differentiate into specific cell lineage pathways prior to, and/or at the time of, their implantation for the therapeutic treatment of elective procedures or pathologic conditions in humans and other species. The use of both autologous and allogenic MSCs is contemplated in this invention.

The investigations reported here confirm the *in vitro* and *in vivo* osteogenic potential of MSCs; demonstrate the *in vivo* osteogenic potential of MSCs when implanted at an ectopic subcutaneous site; and illustrate that purified, culture-expanded MSCs can regenerate a segmental bone defect which would otherwise result in a clinical non-union. These experiments compared the healing potential of MSCs delivered in an osteoconductive, osteoinductive or other appropriate resorbable medium. We also show *de novo* formation of bone at the site of a desired fusion, *e*.*g*. spinal or joint fusions.

The invention provides a method for augmenting bone formation in an individual in need thereof by administering isolated human mesenchymal stem cells with a matrix which supports the differentiation of such stem cells into the osteogenic lineage to an extent sufficient to generate bone formation therefrom. The matrix is preferably selected from a ceramic and a resorbable biopolymer. The ceramic can be in particulate form or can be in the form of a structurally stable, three dimensional implant. The structurally stable, three dimensional implant can be, for example, a cube, cylinder, block or an appropriate anatomical form. The resorbable biopolymer is a gelatin, collagen or cellulose matrix, can be in the form of a powder or sponge, and is preferably a bovine skin-derived gelatin.

Particularly, the invention provides a method for effecting the repair or regeneration of bone defects in an animal or individual in need thereof. Such defects include, for example, segmental bone defects, non-unions, malunions or delayed unions, cysts, tumors, necroses or developmental abnormalities. Other conditions requiring bone augmentation, such as joint reconstruction, cosmetic reconstruction or bone fusion, such as spinal fusion or joint fusion, are treated in an individual by administering, for example into the site of bone in need of augmentation, fresh whole marrow and/or isolated human mesenchymal stem cells or combinations thereof in the gelatin, cellulose or collagen based medium to an extent sufficient to augment bone formation therefrom. The composition can also contain one or more other components which degrade, resorb or remodel at rates approximating the formation of new tissue.

The invention also contemplates the use of other extracellular matrix components, along with the cells, so as to achieve osteoconduction or osteoinduction. In addition, by varying the ratios of the components in said biodegradable matrices, surgical handling properties of the cell-biomatrix implants can be adjusted in a range from a dimensionally stable matrix, such as a sponge or film, to a powder.

The above method can further comprise administering to the individual at least one bioactive factor which induces or accelerates the differentiation of mesenchymal stem cells into the osteogenic lineage. The MSCs can be contacted with the bioactive factor *ex vivo* and are preferably contacted with the bioactive factor when the MSCs are in contact with the matrix. The bioactive factor can be, for example, a synthetic glucocorticoid, such as dexamethasone, or a bone morphogenic protein, such as BMP-2, BMP-3, BMP-4, BMP-6 or BMP-7. The bone morphogenic protein can be in a liquid or semi-solid carrier suitable for intramuscular, intravenous, intramedullary or intra-articular injection.

The invention further provides a composition for augmenting bone formation, which composition comprises a matrix selected from the group consisting of absorbable gelatin, cellulose and collagen in combination with at least one of fresh bone marrow and/or isolated mesenchymal stem cells. The composition can be used in the form of a sponge, strip, powder, gel or web. The invention also provides a method for augmenting bone formation in an individual in need thereof by administering to said individual a bone formation augmenting amount of the composition.

More particularly, the invention provides a method for effecting the repair of segmental bone defects, non-unions, malunions or delayed unions in an individual in need thereof by administering into the bone defect of said person isolated human mesenchymal stem cells in a porous ceramic carrier, thereby inducing the differentiation of such stem cells into the osteogenic lineage to an extent sufficient to generate bone formation therefrom. Preferably, the porous ceramic carrier comprises hydroxyapatite and, more preferably, the porous ceramic carrier further comprises β-tricalcium phosphate. The porous ceramic carrier may also contain one or more other biodegradable carrier components which degrade, resorb or remodel at rates approximating the formation of new tissue extracellular matrix or normal bone turnover.

The invention also provides for the use of other extracellular matrix components, or other constituents, so as to achieve osteoconductive or osteoinductive properties similar to natural extracellular matrix. The composition is an absorbable gelatin, cellulose and/or collagen-based matrix in combination with bone marrow and/or isolated mesenchymal stem cells. The composition can be used in the form of a sponge, strip, powder, gel, web or other physical format. The composition is, for example, inserted in the defect and results in osteogenic healing of the defect.

In addition, by varying the ratios of the components in said biodegradable matrices, surgical handling properties of the cell-biomatrix implants can be adjusted in a range from a porous ceramic block or a moldable, putty-like consistency to a pliable gel or slurry.

More particularly, the invention comprises a rigid cell-matrix implant for large segmental defects, spinal fusions or non-unions, gel or slurry cell-matrix implants, or infusions for stabilized fractures and other segmental bone defects. Custom cell-matrix implants containing autologous or allogeneic MSCs can be administered using open or arthroscopic surgical techniques or percutaneous insertion, *e*.*g*. direct injection, cannulation or catheterization.

In a preferred embodiment, a composition of human mesenchymal stem cells (hMSCs) is obtained from either homogeneous, culture-expanded preparations derived from whole-marrow (or other pre-natal or post-natal source of autologous or allogeneic hMSCs), or from enriched or heterogenous cultures containing an effective dose of hMSCs. The key to effective clinical outcomes using MSC therapy is to provide that number of mesenchymal stem cells to the patient which repairs the bone or other tissue defect. This is referred to as the "Regenerative MSC Threshold", or that concentration of MSCs necessary to achieve direct repair of the tissue defect. The Regenerative MSC Threshold will vary by: 1) type of tissue (*i.e*., bone, cartilage, ligament, tendon, muscle, marrow stroma, dermis and other connective tissue); 2) size or extent of tissue defect; 3) formulation with pharmaceutical carrier; and 4) age of the patient. In a complete medium or chemically defined serum-free medium, isolated, culturally-expanded hMSCs are capable of augmenting bone formation. In an osteoconductive or other optimized medium, such as a resorbable biopolymer, fresh whole bone marrow containing about 10⁴ MSCs per ml of marrow is also capable of augmenting bone formation. Combinations of these techniques are also contemplated.

In another aspect the invention contemplates the delivery of (i) isolated, culture-expanded, human mesenchymal stem cells; (ii) freshly aspirated bone marrow; or (iii) their combination in a carrier material or matrix to provide for improved bone fusion area and fusion mass, when compared to the matrix alone. Particularly preferred is the delivery of a composition comprising purified mesenchymal stem cells and fresh bone marrow aspirates delivered in a carrier material or matrix to provide for improved bone fusion area and fusion mass.

One composition of the invention is envisioned as a combination of materials implanted in order to effect bone repair, osseous fusion, or bone augmentation. The components of this implanted material include, in part, porous granular ceramic, ranging in size from 0.5 mm to 4 mm in diameter, with a preferred size ranging from 1.0 to 2.5 mm in diameter. The composition of the ceramic may range from 100% hydroxyapatite to 100% tricalcium phosphate, and in the preferred form, consists of a 60/40 mixture of hydroxyapatite and tricalcium phosphate. The ceramic material may be uncoated, or coated with a variety of materials including autologous serum, purified fibronectin, purified laminin, or other molecules that support cell adhesion. The granular ceramic material can be combined with MSCs ranging in concentration from 10 thousand to 30 million cells per cc of ceramic, with a preferred range between 3 and 15 million cells per cc. It is also envisioned that the cells may be in the form of fresh marrow obtained intraoperatively, without *ex vivo* culture-expansion.

Bone marrow cells may be obtained from iliac crest, femora, tibiae, spine, rib or other medullary spaces. Other sources of human mesenchymal stem cells include embryonic yolk sac, placenta, umbilical cord, periosteum, fetal and adolescent skin, and blood. The cells are incubated at 37°C with the ceramic for 0 to 5 hours, preferably 3 hours. Prior to implant, the cell-loaded granules can be combined with either fresh peripheral blood, human fibrin, fresh bone marrow, obtained by routine aspiration, or other biological adjuvant. These final combinations are allowed to form a soft blood clot which helps to keep the material together at the graft site. Implant or delivery methods include open or arthroscopic surgery and direct implant by injection, *e*.*g*. syringe or cannula. Finally, these implants may be used in the presence or absence of fixation devices, which themselves may be internally or externally placed and secured.

The composition can also contain additional components, such as osteoinductive factors. Such osteoinductive factors include, for example, dexamethasone, ascorbic acid-2-phosphate, β-glycerophosphate and TGF superfamily proteins, such as the bone morphogenic proteins (BMPs). The composition can also contain antibiotic, antimycotic, antiinflammatory, immunosuppressive and other types of therapeutic, preservative and excipient agents.

### Brief Description of the Drawings

Figures 1A-1D. Phase contrast photomicrographs of rat MSC cultures at various stages of development.
Figure 1A. A MSC colony at day seven of primary culture is composed of uniformly spindle-shaped cells.
Figure 1B. Passage one rat MSCs are distributed evenly across the surface of the dish 4 days after replating.
Figure 1C. Rat MSCs grown in Control Medium for twenty-eight days become confluent and multi-layered, but do not form mineralized nodules. APase staining (dark gray) reveals a fraction of cells which are positive.
Figure 1D. Rat MSC cultures grown in the presence of Osteogenic Supplements for twenty-eight days form mineralized nodules which stain black by the von Kossa method. Cell cultures were stained by APase and von Kossa histochemical techniques as described below (Unstained (a,b), Alkaline phosphatase histochemistry and von Kossa (c,d), all x45).
Figure 2. Light micrograph of a representative histological section from a MSC-loaded HA/TCP implant placed ectopically in subcutaneous tissue. MSCs were loaded and the sample was implanted as described below, harvested at eight weeks, decalcified, and processed in paraffin for microscopy. Only remnants of the HA/TCP ceramic (c) remain, while the pores of the implant are filled with bone (b), blood vessels (v), and hematopoietic elements including adipocytes (Toluidine blue-O, x70).
Figure 3A-3H. High resolution radiographs showing the healing of the segmental defect at four and eight weeks with various implants. The radiographs were obtained on a Faxitron imaging system immediate following sacrifice. The polyethylene fixation plate is on the top of the bone in each radiograph. The four week radiograph is on the left, and the eight week radiograph is on the right for each group. The radiodensity of the HA/TCP material reveals the porous nature and the central canal of each implant.

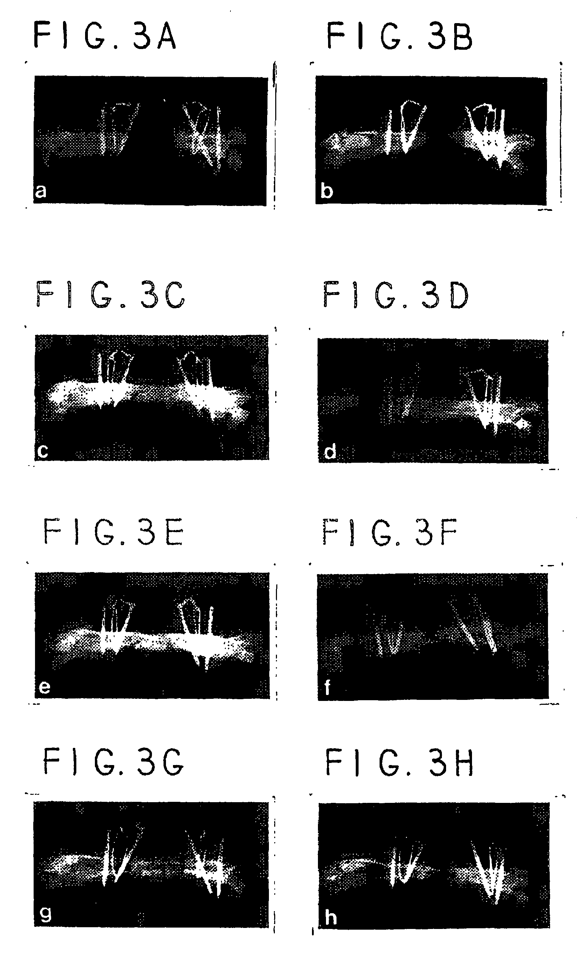

Figures 3A and 3B. Defects left empty;
Figures 3C and 3D. Defects fitted with HA/TCP carrier alone;
Figures 3E and 3F. Defects fitted with a MSC-loaded HA/TCP carrier;
Figures 3G and 3H. Defects fitted with a marrow-loaded HA/TCP carrier. Defects left empty following segmental gap resection undergo reactive bone formation at the cut ends of the bone, leading to a classical non-union in this well established model. At four weeks, the MSC-loaded samples have begun to fill the pores of the implant material. No union is evident in any implant type at four weeks. By eight weeks, modest union of the host-implant interface has occurred in the carrier (d) and carrier plus marrow groups (h), but complete integration and bone bridging is evident in the carrier plus MSC group (f). Total filling of the pores with bone in the MSC-loaded sample is also evident in panel *F*. (x1.5)

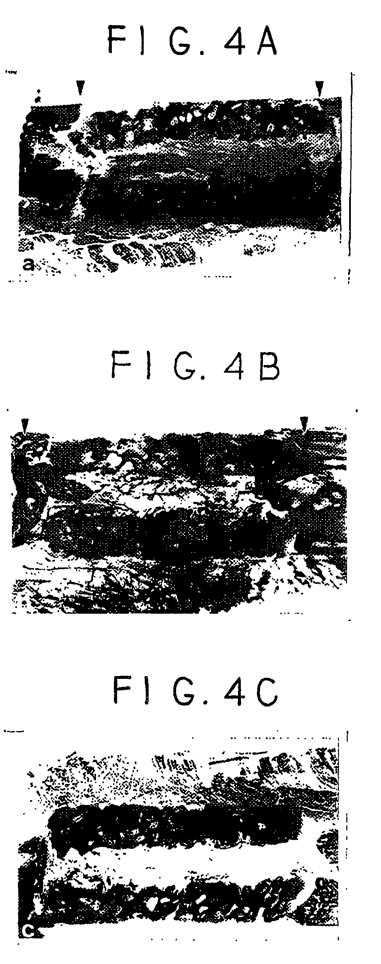

Figure 4A-4F. Light micrographs showing representative healing of the segmental defect at four and eight weeks with various implant types. Intact limbs were harvested, fixed, dehydrated, cleared, embedded in polymethylmethacrylate, cut, and ground to 100 micron thickness prior to staining. Some animals received India ink injections to allow visualization of the vascular tree, present here in panels *B, C*, *D*, and *E* as black staining. The HA/TCP material artifactually appears black in these photomicrographs as a result of undecalcified processing. The cut edges of the host cortices are noted by arrowheads in *a* and *b*, and similar sections are presented in all other panels.
Figures 4A and 4B. Defects fitted with HA/TCP carrier alone at four and eight weeks, respectively;

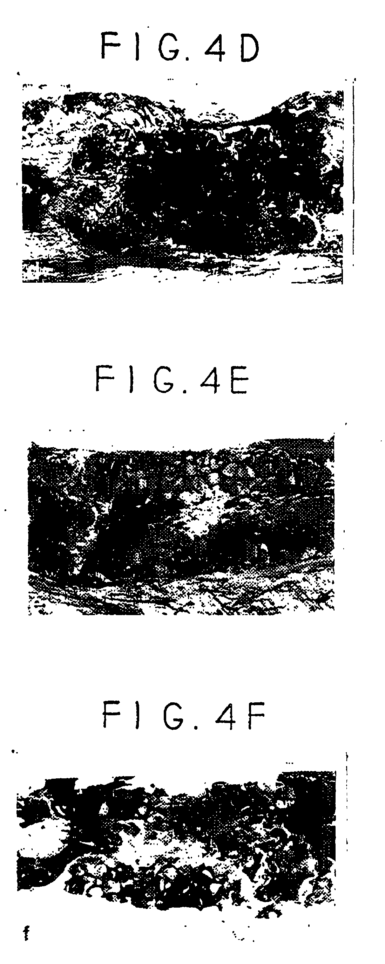

Figures 4C and 4D. Defects fitted with a MSC-loaded HA/TCP carrier at four and eight weeks, respectively;
Figures 4E and 4F. Defects fitted with a marrow-loaded HA/TCP at four and eight weeks, respectively. New bone present within the pores, or at the host-implant interface appears blue or violet in these specimens. Importantly, only samples containing a MSC-loaded implant effectively heal the defect, as noted by the substantial amount of bone present within the implant and at the interface with the host in panels *c* and *d*. See text for further details (Toluidine blue-O, x8).

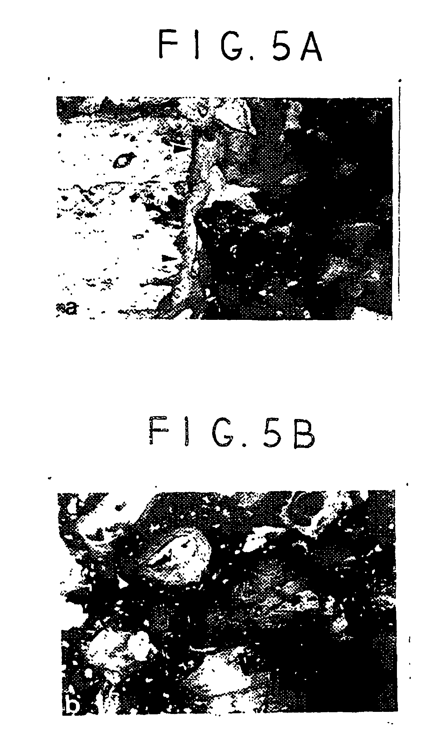

Figure 5A-5B. High power light micrographs showing bone regeneration at eight weeks in segmental gaps fitted with a MSC-loaded HA/TCP implant. Panel *a* shows the cut edge (arrowheads) of the host cortex with new bone in direct apposition. New bone at this host-implant interface is contiguous with bone formed in the pores of the HA/TCP carrier. Panel *b* shows both lamellar and woven bone (blue) filling the pores of the HA/TCP carrier. The carrier appears black in these images as an artifactual result of undecalcified specimen preparation. Blood vessels (v) which orient the secretory activity of osteoblasts are evident within the pores (Toluidine blue-O, x75).

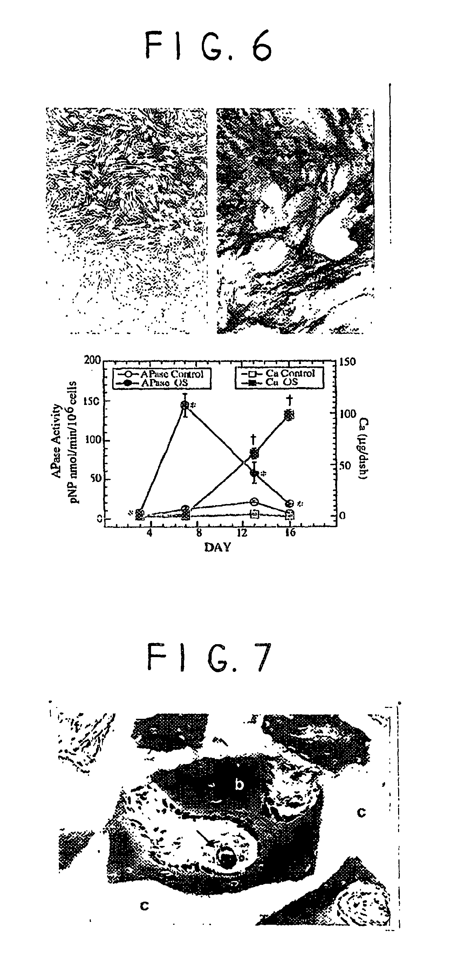

Figure 6. Osteogenic differentiation of human MSCs in vitro. Phase contrast photomicrographs (*a, b*) of human MSC cultures under growth and osteogenic conditions.
Figure 6A. First-passage MSCs display characteristic spindle-shaped morphology and are distributed evenly across the surface of the dish after replating.
Figure 6B. MSC cultures grown in the presence of OS for 16 days form mineralized nodular aggregates which stain gray for APase and black for mineralized matrix (Unstained (*a*) x18, APase and von Kossa histochemistry (*b*), x45).
Figure 6C. APase activity and calcium deposition in MSC cultures grown in Control or OS Medium on days 4, 8, 12 and 16. Samples were harvested at the indicated days, and APase activity, cell number, and calcium deposition were determined as described in Materials and Methods. The results represent the mean ± SD of triplicate cultures from first passage cells. *P<0.05, †P<0.005 (compared to Control).
Figure 7. Light micrograph of a representative histological section from a human MSC-loaded HA/TCP implant placed ectopically in subcutaneous tissue of an athymic rat. MSCs were loaded into the ceramic, implanted as described in Materials and Methods, harvested at 12 weeks, decalcified and processed in paraffin for microscopy. Only remnants of the HA/TCP ceramic (c) remain, while the pores of the implant are filled with bone (b), blood vessels (arrow) or fibrous tissue (f). Cuboidal osteoblasts are seen lining the surface of the developing bone. (Toluidine blue-O, x75).

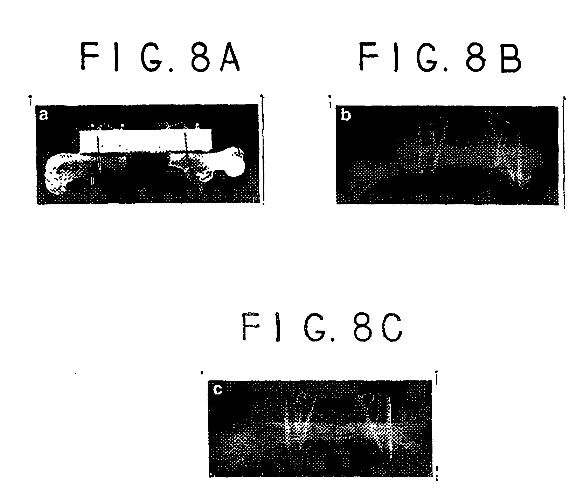

Figure 8. Segmental gap defect model and radiography. (*a*) A polyethylene fixation plate is positioned on the lateral aspect of this representative rat femur. Four bicortical screws and 2 cerclage wires are used to secure the plate in place. An 8 mm segment of bone is removed along with its adherent periosteum, and a ceramic implant, with or without cells, is placed into the defect site. The overlying muscles are returned to their proper anatomic position, and the skin is closed with resorbable sutures. High resolution radiographs obtained immediately following sacrifice show the extent of healing of the segmental defect at 12 weeks with the 2 implant types (*b*, *c*). While total integration of the implant at the host-ceramic interface is evident in the carrier plus MSC group (*b*), only modest union is observed in the cell-free implants (*c*). The pores of the MSC-loaded implant are filled with bone throughout the gap, but the cell-free carrier contains little bone and several cracks.

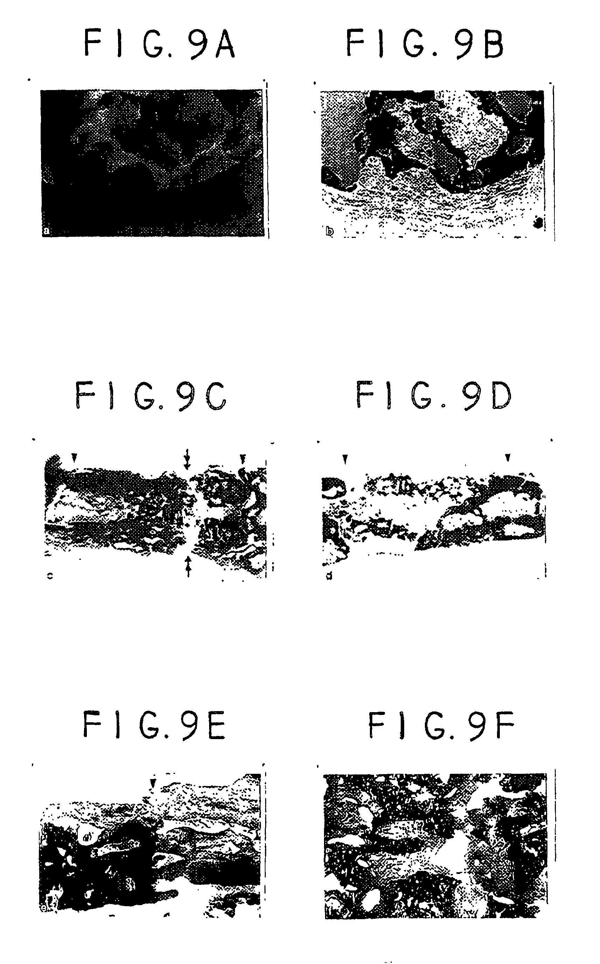

Figure 9. Histologic representation of bone regeneration in segmental femoral defects. Immunohistochemical staining with antibody 6E2 (*a*) demonstrates that 4 weeks following implantation of a MSC-loaded sample, the cells within the pores of the carrier are reactive on their surface, and therefore of human origin, while cells outside the ceramic are not immunoreactive. In phase contrast microscopy (*b*), the ceramic is black, and cells in the pores and surrounding the outside of the implant are evident. The ceramic material itself adsorbs fluorescent secondary antibody and appears green (*a*, *b*, x75). Light micrographs showing representative healing of a segmental defect implanted with HA/TCP carrier alone (*d*), or carrier plus MSCs (*c,e,f*), 12 weeks after implantation. Limbs were harvested, fixed, dehydrated, cleared, embedded in polymethylmethacrylate, cut, and ground to a thickness of 100 µm for staining. The ceramic appears black in these photomicrographs as an artifact of undecalcified specimen preparation, and bone present within the pores or at the host-implant interface appears blue-violet. The MSC-loaded specimen shown here was subjected to destructive mechanical torsion testing, and was subsequently processed for histology in two separate pieces. Repositioning photomicrographs of the two pieces approximates the appearance of the femur prior to testing (*c*). The actual fracture plane is denoted by the double arrows above and below the implant. The cut edges of the host cortices are noted by arrowheads in *c*, *d,* and *e*. Only samples containing a MSC-loaded implant effectively heal the defect. Higher power micrographs demonstrate the substantial amount of bone present at the host-implant interface (*e*) and within the body of the implant (*f*). (Toluidine blue-O, (*c, d*) x7, (*e*) x31, (*f*) x45).

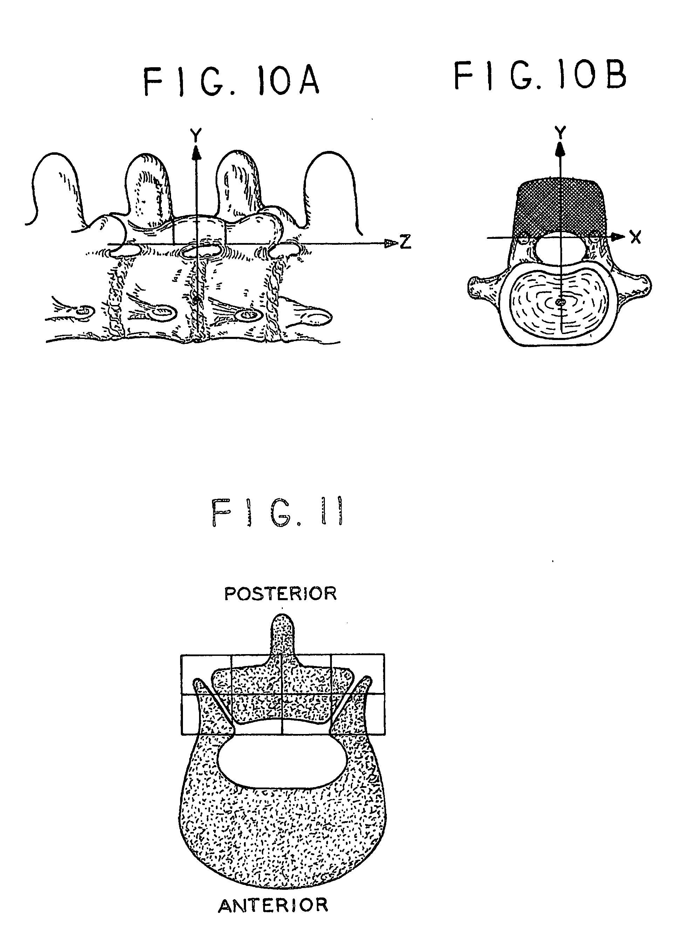

Figures 10A-10B. 3-D coordinate system, defined by the right-hand rule, will be centered at the midpoint of the line between the neural foramen markers at the level of interest, which defines the X axis. The Z direction is defined by a line through the midpoints of the lines between neural foramens at the levels above and below the level of interest (i.e. between orange points). The positive Z direction is cervical to lumbar. The positive Y direction is dorsal. The base of the volume of interest is defined by the X-Z plane. The volume of the fusion mass is defined by the outline of all bone density voxels in the positive Y axis of the X-Y plane between z = -7.5 mm to z = +7.5 mm. Complete fusions will measure 20-30 mm wide in the X-dimension and 10-15 mm in the height in the Y-dimension.
Figure 11. The grid system used to assign union score is shown in schematic cross-section at the level of the facet joints. One half point is assigned for union in each of the box areas.

### Detailed Description of the Invention

Bone grafting procedures are widely used to treat acute fractures, fracture non-unions, bone defects, and to achieve therapeutic arthrodesis. Autogenous cancellous bone is the current "gold standard" for clinical bone grafting. Contemporary dogma attributes this effectiveness to three primary intrinsic properties: osteoconduction, osteogenic cells, and osteoinduction (76,96), which can be defined as follows:
Osteoconduction - The scaffold function provided by the transplanted extracellular bone matrix which facilitates cell attachment and migration, and therefore the distribution of a bone healing response throughout the grafted volume. This property is likely dependent on adhesion molecules within bone matrix such as: collagens, fibronectin, vitronectin, osteonectin, osteopontin, osteocalcin, proteoglycans and others. Growth factors in the matrix may also play a role.
Osteogenic cells - Those cells in the autograft derived from bone or bone marrow which survive transplantation and go on to proliferate and/or undergo osteoblastic differentiation.
Osteoinduction - The bioactive property of autogenous bone derived from the presence of growth factors or other elements in the graft which stimulate the proliferation and/or differentiation of osteoblastic progenitors. Many growth factors have been identified in bone matrix including: bone morphogenetic proteins (BMPs), transforming growth factor-beta (TGF-β), basic fibroblast growth factor (bFGF), and insulin-like growth factor (IGF). Transplanted non-osteogenic cells in bone marrow may also elaborate factors which contribute to a bone healing response (140,48) endothelial cells have been specifically implicated (141).

The marrow or isolated mesenchymal stem cells can be autologous, allogeneic or from xenogeneic sources, and can be embryonic or from post-natal sources. Bone marrow cells may be obtained from iliac crest, femora, tibiae, spine, rib or other medullary spaces. Other sources of human mesenchymal stem cells include embryonic yolk sac, placenta, umbilical cord, periosteum, fetal and adolescent skin, and blood. In order to obtain mesenchymal stem cells, it is necessary to isolate rare pluripotent mesenchymal stem cells from other cells in the bone marrow or other MSC source.

The present invention provides a composition for the repair of bone defects by the rapid regeneration of healthy bone. The composition is an absorbable gelatin, cellulose and/or collagen-based matrix in combination with bone marrow and/or isolated mesenchymal stem cells. The composition can be used in the form of a sponge, strip, powder, gel, web or other physical format. The composition is, for example, inserted in the defect and results in osteogenic healing of the defect.

The composition can also contain additional components, such as osteoinductive factors. Such osteoinductive factors include, for example, dexamethasone, ascorbic acid-2-phosphate, β-glycerophosphate and TGF superfamily proteins, such as the bone morphogenic proteins (BMPs). The composition can also contain antibiotic, antimycotic, antiinflammatory, immunosuppressive and other types of therapeutic, preservative and excipient agents.

The invention also provides a method for treating a bone defect in an animal, particularly a mammal and even more particularly a human, in need thereof which comprises administering to the bone defect of said animal a bone defect-regenerative amount of the composition of the invention.

The investigations reported here confirm the in *vivo* healing potential of fresh whole marrow or MSCs delivered in the matrix alone, or in the matrix in combination.

The invention also contemplates the use of other extracellular matrix components, along with the cells , so as to achieve osteoconductive or osteoinductive properties. In addition, by varying the ratios of the components in said biodegradable matrices, surgical handling properties of the cell-biomatrix implants can be adjusted in a range from a dimensionally stable matrix, such as a sponge or film, to a moldable, putty-like consistency to a pliable gel or slurry to a powder.

The marrow or isolated mesenchymal stem cells can be autologous, allogeneic or from xenogeneic sources, and can be embryonic or from post-natal sources. Bone marrow cells may be obtained from iliac crest, femora, tibiae, spine, rib or other medullary spaces. Other sources of human mesenchymal stem cells include embryonic yolk sac, placenta, umbilical cord, periosteum, fetal and adolescent skin, and blood. In order to obtain mesenchymal stem cells, it is necessary to isolate rare pluripotent mesenchymal stem cells from other cells in the bone marrow or other MSC source.

In a particularly preferred embodiment, the composition of the invention comprises an absorbable implant, containing whole marrow and/or isolated MSCs for repair of segmental defects, spinal fusions or non-unions and other bone defects. Custom cell-matrix implants containing autologous, allogeneic or xenogeneic bone marrow and/or MSCs can be administered using open surgical techniques, arthroscopic techniques or percutaneous injection.

Human mesenchymal stem cells (hMSCs) can be provided as either homogeneous, culture-expanded preparations derived from whole-marrow (or other pre-natal or post-natal source of autologous or allogeneic hMSCs), from hMSC-enriched or heterogenous cultures or fresh, whole marrow (when combined with an osteoinductive or other optimized medium) containing an effective dose of at least about 10', preferably about 10⁴, MSCs per milliliter of the composition. The key to effective clinical outcomes, in this embodiment using MSC therapy, is to provide that number of enriched or culture-expanded mesenchymal stem cells to the patient, or about the same number in an optimized medium, which repairs the bone or other tissue defect beyond that in a volume of whole marrow equivalent to that of the defect. This is referred to as the "Regenerative MSC Threshold", or that concentration of MSCs necessary to achieve direct repair of the tissue defect. The Regenerative MSC Threshold will vary by: 1) type of tissue (*i*.*e*., bone, cartilage, ligament, tendon, muscle, marrow stroma, dermis and other connective tissue); 2) size or extent of tissue defect; 3) formulation with pharmaceutical carrier; and 4) age of the patient.

In a preferred embodiment, the method further comprises administering at least one bioactive factor which further induces or accelerates the differentiation of such mesenchymal stem cells into the osteogenic lineage. Preferably, the cells are contacted with the bioactive factor *ex vivo*, while in the matrix, or injected into the defect site at or following the implantation of the composition of the invention. It is particularly preferred that the bioactive factor is a member of the TGF-β superfamily comprising various tissue growth factors, particularly bone morphogenic proteins, such as at least one selected from the group consisting of BMP-2, BMP-3, BMP-4, BMP-6 and BMP-7.

In the embodiment which uses a gelatin-based matrix, an appropriate absorbable gelatin sponge, powder or film is cross-linked gelatin, for example, Gelfoam^{®} (Upjohn, Inc., Kalamazoo, MI) which is formed from denatured collagen. The absorbable gelatin-based matrix can be combined with the bone reparative cells and, optionally, other active ingredients by soaking the absorbable gelatin sponge in a cell suspension of the bone marrow and/or MSC cells, where the suspension liquid can have other active ingredients dissolved therein. Alternately, a predetermined amount of a cell suspension can be transferred on top of the gelatin sponge, and the cell suspension can be absorbed.

In the embodiment which uses a cellulose-based matrix, an appropriate absorbable cellulose is regenerated oxidized cellulose sheet material, for example, Surgicel^{®} (Johnson & Johnson, New Brunswick, NJ.) which is available in the form of various sized strips or Oxycel^{®} (Becton Dickinson, Franklin Lakes, NJ) which is available in the form of various sized pads, pledgets and strips. The absorbable cellulose-based matrix can be combined with the bone reparative cells and, optionally, other active ingredients by soaking the absorbable cellulose-based matrix in a cell suspension of the bone marrow and/or MSC cells, where the suspension liquid can have other active ingredients dissolved therein. Alternately, a predetermined amount of a cell suspension can be transferred on top of the cellulose-based matrix, and the cell suspension can be absorbed.

In the embodiment which uses a collagen-based matrix, an appropriate resorbable collagen is purified bovine corium collagen, for example, Avitene^{®} (MedChem, Woburn, MA) which is available in various sizes of nonwoven web and fibrous foam, Helistat^{®} (Marion Merrell Dow, Kansas City, MO) which is available in various size sponges or Hemotene^{®} (Astra, Westborough, MA) which is available in powder form. The resorbable collagen-based matrix can be combined with the bone reparative cells and, optionally, other active ingredients by soaking the resorbable collagen-based matrix in a cell suspension of the bone marrow and/or MSC cells, where the suspension liquid can have other active ingredients dissolved therein. Alternately, a predetermined amount of a cell suspension can be transferred on top of the collagen-based matrix, and the cell suspension can be absorbed.

The above gelatin-based, cellulose-based and collagen-based matrices may, optionally, possess hemostatic properties.

Preferred active ingredients are those biological agents which enhance wound healing or regeneration of bone, particularly recombinant proteins. Such active ingredients are present in an amount sufficient to enhance healing of a wound, *i*.*e*., a wound healing-effective amount. The actual amount of the active ingredient will be determined by the attending clinician and will depend on various factors such as the severity of the wound, the condition of the patient, the age of the patient and any collateral injuries or medical ailments possessed by the patient. Generally, the amount of active ingredient will be in the range of about 1 pg/cm² to 5 mg/cm².

The present invention provides a method for augmenting bone formation in an individual in need thereof by administering to said individual isolated human mesenchymal stem cells with a medium which supports the differentiation of such stem cells into the osteogenic lineage to an extent sufficient to generate bone formation therefrom. The medium may be a porous ceramic or resorbable biopolymer. The ceramic may be selected from the group consisting of hydroxyapatite, β-tricalcium phosphate and combinations thereof, may be in particulate form, or may be a structurally stable, three dimensional implant, wherein the structurally stable, three dimensional implant may be a cube, cylinder, block or in the shape of an anatomical form. The resorbable biopolymer may be selected from the group consisting of gelatin, collagen and cellulose. In this embodiment, the medium may be a powder, sponge, strip, film, gel or web or a structurally stable, three dimensional implant in the form of a cube, cylinder or block or in the shape of an anatomical form, or the gelatin may be a bovine skin-derived gelatin. The method of the invention in one embodiment further comprises administering to said individual at least one bioactive factor which induces or accelerates the differentiation of such mesenchymal stem cells into the osteogenic lineage. In one embodiment, the cells are contacted with the bioactive factor *ex viva.*
Preferably, the cells are contacted with the bioactive factor when in contact with the matrix which supports the differentiation of such stem cells into the osteogenic lineage to an extent sufficient to generate bone formation therefrom. In one embodiment, the bioactive factor is a synthetic glucocorticoid, preferably dexamethasone, or a bone morphogenic protein. In one embodiment, the bone morphogenic protein is in a liquid or semi-solid carrier suitable for intramuscular, intravenous, intramedullary or intra-articular injection. In one embodiment, the bone morphogenic protein is selected from the group consisting of BMP-2, BMP-3, BMP-4, BMP-6 and BMP-7.

The present invention further provides a composition for augmenting bone formation, which composition comprises a porous ceramic in combination with at least one of fresh bone marrow and isolated mesenchymal stem cells. The porous ceramic may be in particulate form or may be a structurally stable, three dimensional implant.

The present invention further provides a composition for augmenting bone formation, which composition comprises a resorbable biopolymer selected from the group consisting of gelatin, cellulose and collagen in combination with at least one of fresh bone marrow and isolated mesenchymal stem cells. The resorbable biopolymer may be in particulate form or may be a sponge, strip, film, gel or web or a structurally stable, three dimensional implant.

The present invention further provides a method for augmenting bone formation in an individual in need thereof which comprises administering to said individual a bone formation augmenting amount of a composition for augmenting bone formation, which composition comprises a porous ceramic in combination with at least one of fresh bone marrow and isolated mesenchymal stem cells. The porous ceramic may be in particulate form or may be a structurally stable, three dimensional implant.

The present invention further provides a method for augmenting bone formation in an individual in need thereof which comprises administering to said individual a bone formation augmenting amount of a composition for augmenting bone formation, which composition comprises a resorbable biopolymer selected from the group consisting of gelatin, cellulose and collagen in combination with at least one of fresh bone marrow and isolated mesenchymal stem cells. The resorbable biopolymer may be in particulate form, or may be a sponge, strip, film, gel or web or a structurally stable, three dimensional implant.

### Example 1

### Rat Gap Defect Repair

### Materials & Methods

### Materials

Dexamethasone (Dex), sodium β-glycerophosphate (βGP), antibiotic penicillin/streptomycin, and alkaline phosphatase histochemistry kit #85 were purchased from Sigma Chemical Co. (St. Louis, MO), DMEM-LG (DMEM) tissue culture medium from GIBCO Laboratories (Grand Island, NY), and L-ascorbic acid-2-phosphate (AsAP) from Wako Chemical (Osaka, Japan). Fetal bovine serum (FBS) was purchased from GIBCO following an extensive testing and selection protocol (80). Porous hydroxyapatite/β-tricalcium phosphate (HA/TCP) ceramic, mean pore size 200-450 µm, was generously provided by Zimmer, Inc. (Warsaw, IN). All other routine reagents used were of analytical grade.

### MSC Isolation and Cultivation

MSC isolation and culture expansion was performed according to previously published methods (32). Briefly, male Fisher F344 rats (200-275 g) were sacrificed by pentobarbital overdose. The tibias and the femurs were recovered by dissection under sterile conditions, the metaphyseal ends of the bones were cut, and the marrow plugs were flushed out by passing saline through a needle inserted into one end of the bone. Pooled marrow clots were dispersed by gentle pipetting, followed by sequential passage through a series of smaller needles yielding a single-cell suspension. The cells were then centrifuged for ten minutes at 900 xg, and resuspended in DMEM containing 10% FBS (Control Medium). Fifty million nucleated cells were plated onto petri-dishes (sixty cm²) in seven milliliters of Control Medium, and grown at 37°C in the presence of 5 % CO₂. Non-adherent cells were removed at the time of the first medium change, four days post plating, and cells were routinely fed twice weekly thereafter. These primary cultures approached confluence typically at thirteen days, were then released by a five minute exposure to 0.25% trypsin containing one millimolar EDTA, and subcultivated at a density of 10⁴ cells/cm². Cells for implantation were derived from these first passage cultures ten days after replating, at which time they were approximately 85% confluent.

### In Vitro Osteogenic Assays

At the end of first passage, MSCs were replated into six-well plates at a density of 10⁴ cells/cm² in Control Medium. The following day (Day 0), fresh Control Medium was provided, and the cells were grown in the absence or presence of Osteogenic Supplements (OS) (100 nanomolar Dex, 0.05 millimolar AsAP and ten millimolar β-GP) (64). Media changes were performed twice weekly, and at days seven, fourteen, twenty-one, and twenty-eight, cultures were assayed for cell number, alkaline phosphatase (APase) histochemistry, and mineralized matrix production utilizing techniques previously described (64).

### Implant Preparation

HA/TCP blocks were shaped into cylinders approximately four millimeter in diameter and eight millimeter in length. A central canal roughly one millimeter in diameter was bored through the length of the entire cylinder using an eighteen gauge hypodermic needle. Cylinders were cleaned by sonication and rinsing in distilled water, and then sterilized by 220°C dry heat for five hours. The cylinders were subsequently coated with human plasma fibronectin (Cal-Biochem, Irvine, CA) by soaking in a 100 microgram per milliliter solution for sixteen hours at 4°C. The implants were then air dried at room temperature overnight in a sterile biosafety cabinet, and stored at 4°C. HA/TCP cubes, measuring three millimeter per side, were similarly prepared and coated with fibronectin as described above for use in the ectopic osteogenesis assay.

HA/TCP implants, both in cube and cylinder form, were loaded with MSCs using a modification of a technique previously described (32,83). Briefly, implants were placed in a suspension of MSCs (7.5 x 10⁶ cells/ml) in serum free DMEM. The loading vessel was capped, and the implants were subjected to a vacuum in three bursts of five seconds each to remove air present within the pores of the HA/TCP, and to facilitate fluid flow into the pores. The loading vessels were capped loosely, placed in a tissue culture incubator for two hours, and gently agitated every thirty minutes until the time of surgery. Cell-free control cylinders were treated identically, with the notable exception that the serum free DMEM contained no cells. The third implant group was designed to generously approximate the clinically relevant control of a fresh bone marrow aspirate. Just prior to implantation, fresh marrow cell suspensions were obtained as previously described, centrifuged for ten minutes at 900 xg, and resuspended in a volume of serum free DMEM which would coat each cylinder with the number of bone marrow cells derived from one entire femur, approximately fifty million (144,145). The HA/TCP implants were loaded with this fresh marrow by rolling them in the congealed marrow suspension.

### Surgical Model and Experimental Design

The rat femoral gap model described here is a modification of one used extensively to study long bone repair (34,37,61,83,105,126,129,144,145,147). Briefly, both femurs of male F344 rats (300-350 g) were exposed by an anterolateral approach. Soft tissue and muscle was elevated while keeping the periosteum intact along the surface of the bone. A polyethylene fixation plate (four by four by twenty-three millimeters) (Hospital for Special Surgery, New York, NY) was secured to the anterolateral aspect of each femur by four threaded Kirschner-wires and two cerclage wires (Zimmer, Warsaw, IN). An eight millimeter transverse segment of the central diaphysis, along with its adherent periosteum, was removed by a rotary osteotomy burr under saline irrigation. These stabilized segmental defects were either left empty, or replaced with a cell-free HA/TCP cylinder, a MSC-loaded cylinder, or a cylinder loaded with a fresh marrow cell suspension. Implants were secured by placing two 4-0 Vicryl (Ethicon, Somerville, NJ) sutures around the ceramic and the fixation plate. The muscles were apposed, and the fascia and skin were closed in a routine layered fashion. Rats implanted with MSC-loaded cylinders also received subcutaneous implants of the MSC-loaded HA/TCP cubes to correlate the ectopic osteogenesis assay with orthotopic bone regeneration and the in vitro osteogenic potential of syngeneic MSCs. Rats implanted with marrow-loaded cylinders similarly received subcutaneous implants of marrow-loaded cubes. The animals were allowed full activity in their cages post-operatively. No animals experienced failure of fixation or other post-operative complications. At least six limbs were used for each of the implant groups, randomly selected between left or right. Upon sacrifice at four and eight weeks, the vascular tree of some animals was perfused with India ink, and the entire femur and surrounding soft tissue was carefully dissected. Specimens were immediately evaluated radiographically, and subsequently processed for undecalcified histology.

### Radiographic Analysis

The specimens were radiographed using a high resolution Faxitron Imaging system (Buffalo Grove, IL) with an exposure of thirty-five kVP for thirty seconds. The radiographs were independently evaluated by two of the authors who were blinded with respect to the duration and type of implant. Bone formation was scored on a semiquantitative scale with ranges as follows: distal host-implant union (0-2); proximal host-implant union (0-2); and implant core density (0-4). The union scores and the core density scores were added to give a maximum possible score of eight for each implant. Results from both examiners were averaged to give final scores.

### Histology and Histomorphometry

Following fixation in 10% buffered formalin, the femurs were dehydrated, cleared, and embedded in polymethylmethacrylate. Longitudinal sections were cut on a water-cooled Isomet saw (Buehler, WI), and a central section of each leg was ground to 100 micrometer thickness, polished, and stained with Toluidine blue-O. Leica Quantimet 500MC (Cambridge, UK) image analysis software was used to determine the area of HA/TCP implant, bone, and soft tissue in the diaphyseal defect region of each section. The data were analyzed by one-way analysis of variance (ANOVA) (Sigmastat, Jandel Scientific). Further analyses were performed according to *post hoc* Student-Newman-Keuls tests. Subcutaneously implanted ceramic cubes were similarly fixed in formalin, then decalcified, dehydrated, embedded in paraffin, serially sectioned, and stained with Toluidine blue-O.

### Results

### MSC Cultivation and Osteogenic Differentiation in vitro

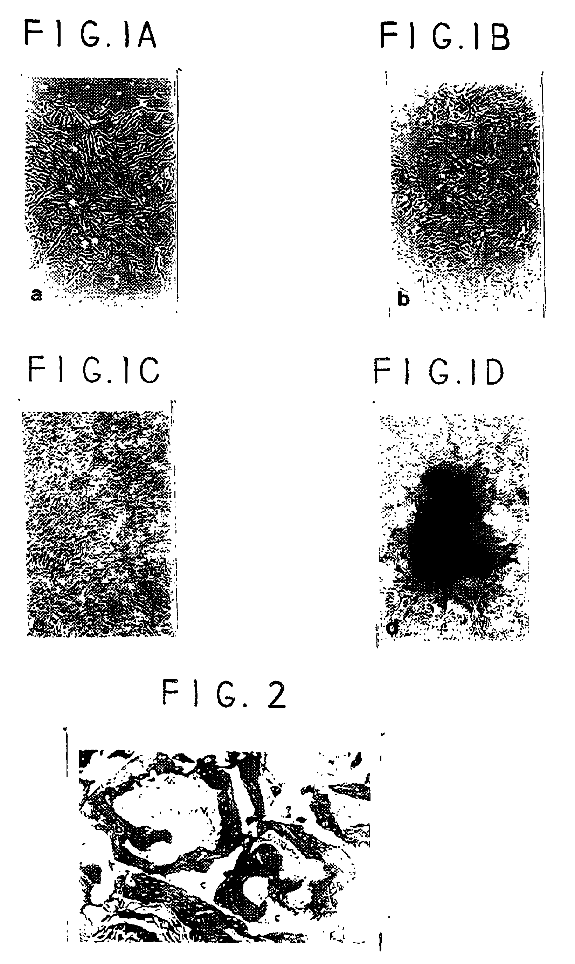

Rat MSC cultures were established from syngeneic animals and, by seven days, formed characteristic colonies on the surface of the culture dish (Fig. 1A). Several hundred MSC colonies arose from the fifty million nucleated cells seeded on each sixty cm² dish. On the basis of this observation, rat MSCs, like human MSCs (13,54), appear to be present at a frequency of approximately one in 10⁵ nucleated marrow cells. Primary MSC cultures subcultivated on day fourteen attached uniformly to the surface of new dishes, and were allowed to divide for roughly ten days, or until the dishes became ∼ 85 % confluent. Passaged cells also demonstrate a characteristic morphology (Fig. 1B), and uniformly divide upon the dish resulting in an even distribution of MSCs throughout the plate. Cells derived from this first passage were used for preparing implants as described above, and an aliquot was used to confirm the *in vitro* osteogenic potential of rat MSCs.

Seven days after replating for the osteogenic assay, both Control and OS-treated cultures were composed of spindle-shaped cells, 40-50% of which were stained for APase. During the next twenty-one days, Control cells remained fibroblastic, increased their cell surface APase, but never underwent the morphologic changes associated with the development of mineralized bone nodules (Fig. 1C). By contrast, OS-treated cultures began to form aggregates of polygonal and cuboidal cells intensely stained for APase, and by day twenty-one, the cultures had formed characteristic bone-like nodules which contained von Kossa stained mineral deposits. Further mineralization of these nodules through day twenty-eight (Fig. 1D) was accompanied by a decrease in APase staining, especially within the internodular regions.

### MSC-Mediated Osteogenesis in Ectopic HA/TCP Implants

All MSC-loaded HA/TCP cubes implanted in the host rats had ample evidence of osteogenesis by four weeks. At the eight week time point, a substantial amount of bone, and occasionally cartilage, was present within the pores of the cubes. A representative section from a MSC-loaded cube harvested eight weeks following implantation is shown in Figure 2. The unstained granular areas reflect the former regions of ceramic material which have been removed during the decalcification step of specimen preparation. As seen in the photomicrograph, bone formation occurs within the pores of the cubes, and is associated with vascular elements which penetrate the implant. Such angiogenesis is obligatory to new bone formation since the secretory activity of osteoblasts is an oriented phenomenon guided by vasculature. Both woven and lamellar bone can be seen depending on the duration of implantation, and the precise region examined. Most of the pores are filled with bone and small islands of hematopoietic elements, with the remainder being filled with a loose connective tissue. In contrast to these MSC-loaded samples, cubes loaded with fresh marrow contained negligible osseous tissue at four weeks, and only slightly more even at eight weeks. As previously demonstrated (32,54), cubes implanted without MSCs or marrow contained no bone, but were filled with fibrous tissue and blood vessels.

### Radiographic Evaluation

High resolution Faxitron radiographs provided sufficient clarity and detail to discern subtle changes occurring within the implant and the surrounding host bone. Figure 3 shows representative radiographs of the femurs from each of the groups recovered at four and eight weeks post-implantation. As demonstrated in these radiographs, the fixation remained intact in all the samples and there were no fractures in any of the femurs. In animals whose femoral defects were left empty, reactive bone formation at the transversely cut edges of the host femur was observed at four weeks (Fig. 3A). By eight weeks, slightly more bone was present within the gap, however, most of this bone appeared to form along the edge of the fixation plate which was in contact with the periosteum (Fig. 3B). Every specimen which was left empty resulted in the formation of a radiographic non-union. Some limbs, irrespective of the group, also contained an eccentric spicule of bone which was usually on the outside of the defect opposite the fixation plate.

When the HA/TCP cylinder was implanted, negligible reactive bone formation occurred at the cut edges of the femur. Due to the mineral content of the implant material (HA/TCP), one can appreciate the structural details of the implant itself upon radiographic evaluation. The details of the central canal and pores are clearly visible in the four week radiographs (Fig. 3C), and serve to provide an important baseline for comparison to the other radiographic images. Blurring of the pore margins can be appreciated by eight weeks (Fig. 3D) in these cell-free implants. Importantly, the lack of union between the implant and the host is manifested as a clear zone of radiolucency between the implant itself and the cut edges of the femur in all animals at four weeks. In contrast to the four week carrier alone, animals which received MSC-loaded HA/TCP cylinders demonstrated substantial new bone formation within the pores of the implant by four weeks (Fig. 3E). Increasing radiodensity, and obliteration of the apparent pore structure, was used as an indication of new bone formation within the core of the implant. Although integration of the implant, or union, was not observed by four weeks, the subsequent formation of a radiodense bone bridge between the implant and the host completely masked the interface. By eight weeks, the MSC-loaded implant was contiguous and completely integrated with the normal host bone (Fig. 3F). HA/TCP implants which were loaded with fresh marrow did not appear to produce radiodense bone within the pores at either time point, although modest integration with the cut ends of the host bone was evident by eight weeks (Figs. 3G and 3H).

The average of the radiographic scores at each time point for each implant group is provided in Table 1.

**Table 1**

| **Average of Radiographic Scores for Each Implant Group** | | | | | | |
|---|---|---|---|---|---|---|
| | Four Weeks | | | Eight Weeks | | |
| | C only | C+MSCs | C+M | C only | C+MSCs | C+M |
| Proximal Union | 1.5 | 0.8 | 0.2 | 1.2 | 1.3 | 1.0 |
| Distal Union | 0.5 | 1.4 | 0.7 | 1.2 | 2.0 | 1.6 |
| Core Density | 0.7 | 2.0 | 0.3 | 0.6 | 3.7 | 0.7 |
| Total Score | 2.7 | 4.2 | 1.2 | 3.0 | 7.0* | 3.3 |

**Table 1.** Average of radiographic scores for each implant group at each time point. C = carrier, M = marrow. Radiographs were evaluated and scored by two independent observers blinded to the identity of each implant. Union was scored both proximally and distally on a scale of 0-2. Core density was scored on a scale of 0-4. n=3 for each group at each time point. The maximum possible total score is 8. One-way analysis of variance at the two different time points, with cell loading (none, MSCs, and marrow) as the independent variable showed significant difference between groups at 8 weeks (F = 10.9, p = 0.01) but were not significantly different at four weeks. * = significantly greater (p < 0.05) than other groups at the corresponding time point (according to *post hoc* Student-Newman-Keuls tests).

In the case of the defects filled with the HA/TCP carrier alone, the low scores indicate the absence of any radiodense material within the pores, and minimal union of the implant with the host bone. Loading the HA/TCP implant with fresh marrow did not result in an improvement in the healing of the defect, and the low scores reflect the similarity of this group to that of the carrier alone. However, loading the HA/TCP carrier with MSCs produces a vigorous osteogenic response. Even at four weeks, pore filling was observed and is reflected in the considerably higher scores of these implants. Interestingly, even in this case the host-implant union was modest compared to controls. By the eight week time point, the pores of the implant were filled with new bone and the host-implant union was well established.

### Histologic and Histomorphometric Evaluation

Histologic evaluation of the samples confirmed the observations made by radiography. In the empty defects, reactive bone formation appeared to emanate from the cut ends of the host cortices and endosteum. Even at eight weeks there was no bridging across the defect, and a fibrous non-union had formed at the center of the segmental gap. Photomicrographs of representative sections of the implant groups recovered at four and eight weeks are shown in Figure 4. In defects fitted with the HA/TCP carrier alone, the pores of the implant were filled with fibrous tissue (Fig. 4A) and were well vascularized as determined by India Ink injection. No bone could be seen within the pores of the implant and there was limited integration with the host. Even at eight weeks, most of the pores were devoid of any bone despite significant vascularization evident in this photomicrograph (Fig. 4B). A small amount of new bone was present at the host-implant interfaces, and at one end of this representative implant, host-derived endosteal bone appears to be advancing into the medullary canal of the implant. Bone formation in samples loaded with fresh marrow was very similar to that of the HA/TCP carrier alone (Figs. 4E and F). However, a modest amount of new bone could be seen within the pores of the implant at eight weeks, correlating with the results of the ectopic implants. Union of these implants was similar to that observed with cell-free implants; reactive bone formation slightly penetrated the pores at the ends of the implant.

In contrast to the sparse osteogenesis resulting from the addition of fresh marrow to the HA/TCP, most of the pores of the implants loaded with MSCs contained considerable new bone by four weeks (Fig. 4C). Again, there was still a clear demarcation between the cut edges of the host bone and the ends of the implant. At eight weeks nearly every pore was filled with new bone, except in some discrete areas where loading of the MSCs may have been suboptimal. Interestingly, substantial new bone formation occurred at the interface between the host and the implant, leading to a continuous span of bone across the defect (Fig. 4D). Furthermore, a periosteal callus was also present in samples loaded with MSCs (Fig. 4D), but not in other implant types. The bone formed within the pores and at the ends of these implants represents *de novo* bone formation, is highly cellular, and is presented in higher magnification photomicrographs in Figure 5. New woven and lamellar bone can be seen in intimate contact with the cut edge of the host cortex at eight weeks (Fig. 5A). Importantly, this region of union is directly contiguous with bone formed throughout the pores of implant. In regions deeper within the HA/TCP, filling of the pores with new bone is evident, as is the association of vasculature which orients the secretory activity of the differentiating osteoblasts (Fig. 5B).

The results qualitatively described above are mirrored in the histomorphometric data presented in Table 2.

**Table 2**

| **Carrier alone** | **Carrier + MSCs** | **Carrier + Marrow** |
|---|---|---|
| 2.3 ± 1.5 | 19.3 ± 3.7* | 2.9 ± 1.7 |
| 10.4 ± 2.4 | 43.3 ± 7.7* | 17.2 * 6.0 |

**Table 2.** Bone fill in HA/TCP implants as a percentage of available space. Histomorphometric measurements were obtained on the bone formed within the confines of the segmental resection, excluding the implant material itself and the medullary canal. The values are reported as means of three samples along with standard deviations from the mean. One-way analysis of variance at the two different time points, with cell loading (none, MSCs, and marrow) as the independent variable showed significant difference between MSC-loaded samples at both 4 weeks (F = 43.3, p < 0.001) and 8 weeks (F = 26.2, p < 0.002). * = significantly greater (p < 0.01) than other groups at the corresponding time points (according to *post hoc* Student-Newman-Keuls tests). No difference was observed between marrow and carrier alone at either time point (p > 0.1).

The cell-free HA/TCP implants had a bone fraction of only 2.3 % and 10.4% at four and eight weeks, respectively. Importantly, this fraction of bone at eight weeks correlates with previously published results (126). These fractions primarily represent the bone ingrowth from the cut ends of the host cortices. The marrow-loaded HA/TCP cylinders did exhibit modest osteogenesis within the body of the implant and consequently had a slightly higher value of 17.2% at eight weeks. Importantly, by four weeks, the MSC-loaded samples exceeded the eight week value for the other two groups. The 19.3% bone fill at this four week time point is most likely attributable to MSC-mediated osteogenesis. The average bone fraction within the implant increased over time, reaching 43 % by eight weeks. One-way ANOVA performed on the data along with the Student-Newman-Keuls tests showed that at both four and eight weeks, the MSC treatment was significantly better than the carrier alone or the marrow-loaded carrier (p < 0.01). No significant difference between carrier alone and marrow-loaded implants was detected. The volume fraction of the HA/TCP carrier remained constant, and served as an internal control for the histomorphometry system. Even though the empty defects had 34% bone fill by eight weeks, there was no bridging across the defect, and thus would be classified as a clinical non-union.

### Discussion

In the present study, we have demonstrated that purified, culture-expanded syngeneic progenitor cells are capable of healing a clinically significant bone defect in a well established animal model. These progenitor cells are referred to as mesenchymal stem cells since they give rise not only to bone (11,32,54,64), but to cartilage (32,66,80,142), muscle (121,143), tendon (23), and a stromal tissue which supports hematopoietic differentiation (87). While the osteogenic potential of both animal and human MSCs has been proven via subcutaneous implants in ectopic assays, rigorous and quantitative studies establishing the ability of culture-expanded MSCs to regenerate large segmental bone defects have not been reported to our knowledge. The combination of MSCs with a porous HA/TCP implant material are shown in the present study to be an effective strategy for healing large segmental bone defects. The current investigation further substantiates that compared to fresh marrow, MSCs produce significantly more bone when placed in either an ectopic or an orthotopic site. With these results as a foundation, we may begin to refine our approach to autologous cell therapies for the regeneration of skeletal defects.

To further characterize the cells used in this study, we cultured them in the presence and absence of a medium which induces osteogenic differentiation *in vitro.* As has been reported in numerous other laboratories (77, 88,89,118,135), these rat marrow-derived cells develop along the osteogenic lineage in response to dexamethasone, eventually forming mineralized nodules of bone-like tissue on the surface of the dish. Such differentiation is evident in our photomicrographs (Fig. 1), and serves to document that the cells used in these implants indeed possess the ability to form bone, one of the inherent properties of MSCs. Furthermore, the bone and cartilage formed in cubes implanted subcutaneously not only confirms the osteochondral potential of the MSCs, but acts as an internal control to verify that every host rat was capable of providing an environment which could support osteogenesis within these combined cell:matrix implants. Additional experiments documenting the multilineage potential of these cells were not included as part of the current study because previous publications have focused on describing such potential in greater detail (32,79,80,121,143). The isolation and selection procedures for rat MSCs are similar to those used for human MSCs (32,54,80), and result in the formation of characteristic primary colonies illustrated in Figure 1A. These cells are mitotically expanded to yield a morphologically homogeneous population which divides uniformly across the dish. Both human and rat MSCs have been shown to possess multilineage potential, and the details of *in vitro* osteogenic differentiation of human MSCs has recently been reported (33,64). Conditions for the isolation and culture expansion of human MSCs without lineage progression have been optimized (13,54,80), and the development of a serum free medium for human MSC growth has been completed (58).

The radiographic findings in this study establish a precedent for obtaining non-invasive evidence of bone regeneration in animals, or humans, which receive MSCs in an orthotopic location. Given the porous nature of the HA/TCP implants, new bone which forms within the interstices of the material is readily apparent radiographically by four weeks, in spite of the inherent radiodensity of the HA/TCP material. The progressive increase in radiodensity evident by eight weeks correlates well with the histological observations of processed limbs. Interestingly, despite the presence of new bone within the core of implants by four weeks, integration at the host-implant interface was not observed until eight weeks. The mean radiographic scores for the three implant groups document a significant (p < 0.05) difference between MSCs and either marrow-loaded and ceramic implants at eight weeks, while no significant difference was observed between marrow-loaded and ceramic implants at either time point.

The histologic studies demonstrate appositional bone growth on the surface of the HA/TCP throughout the core of the implant, consistent with previous observations of osteogenesis in ectopic implants loaded with MSCs (32,47,54). The bone which is formed at four and eight weeks in MSC-loaded samples is woven in many areas, but lamellar bone can also be appreciated (Figs. 5A and 5B). It is critical to note that in the process of regenerating this osseous defect, bone formation occurs by a direct conversion of mesenchymal cells into osteoblasts rather than by an endochondral sequence. As regeneration of the bone at the defect site continues, the pores of the ceramic are filled with significantly more bone, which is laid down upon the walls of the implant or existing bone, and oriented by the invading vasculature. These blood vessels, visualized by India inking of animals immediately prior to sacrifice, also provide a portal for the entry and establishment of new marrow islands which contain hematopoietic elements, as well as host-derived MSCs. The process of bone remodeling ensues, and eventually the donor bone is replaced by host bone (47). At the edge of the defect, integration of the implant is achieved with direct continuity between the cut edge of the host cortex and the new bone formed upon the surface of the implant (Fig. 5A). Since only minimal host-implant union occurs in rats provided with either marrow-loaded or cell-free ceramics, the advanced integration observed in MSC-loaded ceramics likely reflects the combined contributions of implanted MSCs and host-derived cells. The lack of early union in all samples was surprising in light of the fact that defects which were left empty underwent a substantial amount of reactive bone formation at the cut edges of the cortices. It is possible that the presence of an implant in the defect site inhibits migration and/or prolapse of the surrounding loose mesenchyme which contributes to the reactive bone formation in the empty defects. Furthermore, micromotion of the implanted cylinders would likely hinder stable union at the interface.

The ability of MSCs to regenerate a large segmental defect in this experimental model compares favorably with other investigations testing implants such as demineralized bone matrix, bone marrow, purified or recombinant BMPs, allograft, ceramics, and fibermetals (34,37,74,83,105,126,129,144,145,147). While the use of recombinant BMP has received considerable attention, the precise mechanism of action has only recently been appreciated. These powerful inductive molecules act on undifferentiated mesenchymal cells to initiate the endochondral cascade, ultimately resulting in the formation of bone. Studies of undifferentiated rat marrow stromal cells confirm that BMP-2 acts to directly stimulate osteoblast development, and that this stimulation is enhanced by the addition of dexamethasone (77). Others have shown that bone formation occurs in an orthotopic site when fresh marrow alone is added, but the rate and extent of healing is a function of the amount of marrow and the number of osteoprogenitor cells residing therein (26,49,129,144). An important set of experiments by Takagi and Urist (129) demonstrate that the addition of BMP is not effective at healing segmental defects when access to the medullary canal and the marrow stroma is prevented, thus indicating an absolute requirement for the cellular constituents of marrow in BMP-mediated bone repair. These results were bolstered by studies indicating that the implantation of fresh marrow along with BMP in a rat segmental gap model is more effective than either component implanted alone (74). One may conclude from all of the above that marrow-derived mesenchymal progenitors, or MSCs, are the target for endogenous osteoinductive molecules, such as BMPs, which are released during normal bone healing. It therefore follows that one must have an adequate supply of MSCs in order to respond to the normal (or exogenously supplied) signals of bone repair, or healing will be effete.

The histomorphometric data generated in this study provides a basis for comparison to other investigations. When fresh marrow from one femur equivalent is loaded on an HA/TCP implant, no significant difference in bone formation is observed when compared to implants which receive no cells. This is true for both time points in our study, and likely reflects an inadequate number of MSCs in the volume of marrow applied. Had we loaded the implants with considerably more marrow, we and others would predict that greater healing of the bone defect would have occurred (74,144). Nevertheless, an appropriate clinically relevant control is generously approximated by applying the total cell population obtained from one long bone since removing all the marrow from multiple long bones for the repair of a focal defect is contradictory to sound clinical judgment. Perhaps most importantly, MSCs produced a bone fill of 19.3 % and 43.2 % , respectively, at four and eight weeks. When purified BMP was applied to an identical carrier in the same experimental model, the bone fill was 21 % at four weeks, and only 22 % by eight weeks (126). These BMP-coated HA/TCP implants did not achieve a bone fill of 43 percent until 16 weeks following implantation. While similar amounts of bone resulted from both implant types at four weeks, MSCs produce twice as much bone as BMP by the eight week time point. In this formulation, it took BMP sixteen weeks to form the same amount of bone which MSCs produce in only eight weeks. On this basis, it appears that MSCs offer a considerable advantage to the use of BMP alone, although some combination of BMP and MSCs could provide an even faster, more vigorous bone repair as discussed above.

Since the number of progenitor cells present at the site of repair is a critical factor, it is obligatory to estimate how the MSC-loaded implants compare with marrow-loaded implants in this regard. The number of nucleated marrow cells which were placed on an implant was approximately fifty million; the same number harvested from one long bone. Another fifty million cells were used to initiate the MSC culture which eventually provided cells for one implant. From these fifty million cells, roughly 500 MSC colonies develop, and these cells are mitotically expanded to three million by the end of first passage. This represents a 6,000-fold increase in MSC number due to approximately twelve population doublings. Using the current technique to load these type of implants, it appears that only about 150,000 cells become adherent following incubation with the MSC suspension (32). Nevertheless, the local administration of 150,000 purified MSCs would increase the number of progenitor cells 300 times over the number normally present in fifty million unfractionated marrow cells. On the basis of these calculations, the advantage which this technique offers over other bone regeneration strategies is direct delivery of the cellular machinery required for bone formation. This approach would have an extraordinary advantage in settings where the number of endogenous progenitor cells is reduced, such as that which occurs in ageing, osteoporosis, or a variety of other pathologic conditions (33,72,82,118,128,135). Other investigators have pursued this logic by attempting to deliver more progenitor cells simply by concentrating the marrow, by crude fractionation and removal of red blood cells, or by cultivating the stromal cells *in vitro* (26,83,103,105,144). Now that techniques and conditions have been established which support the expansion of purified human MSCs in culture as much as one billion fold without a loss in osteogenic potential (13) analogous clinical protocols for regenerating human bone defects are not far away. It will be possible to further expedite the healing process by directing these culture-expanded MSCs *ex vivo* to enter the osteogenic lineage prior to implantation, thus decreasing the *in situ* interval between implantation and their biosynthetic activity as osteoblasts. Additional efforts are underway to develop cell delivery vehicles which will provide more flexibility to the surgeon, including materials which can be shaped to fit any type of defect. By combining a pharmacologic stimulus, such as BMP, with an even better delivery vehicle, we will be able to offer patients therapeutic options which have never before been available.

### Example 2

### Large Segmental Canine Femoral Defects Are Healed

### With Autologous Mesenchymal Stem Cell Therapy

This study demonstrates that culture-expanded, autologous mesenchymal stem cells can regenerate clinically significant bone defects in a large animal model.

Recently, the ability of syngeneic bone marrow-derived mesenchymal stem cells (MSCs) to repair large segmental defects in rodents was established (68). These MSCs may be isolated from marrow or periosteum, expanded in number *ex vivo*, and delivered back to the host in an appropriate carrier vehicle. Studies in rats demonstrated that the amount of bone formed 8 weeks following implantation of MSCs was twice that resulting from BMP delivered in the same carrier (68,126). In order to demonstrate clinical feasibility of this technology, our objective was to regenerate segmental bone defects in a large animal amenable to stringent biomechanical testing. To achieve this goal, we developed a canine femoral gap model to compare radiographic, histologic, and biomechanical data following implantation of an MSC-loaded carrier, carrier alone, and cancellous autograft bone.

### Materials and Methods

### MSC Cultivation and Manipulation

A 15cc bone marrow aspirate was obtained from the iliac crest of each animal, according to an IACUC-approved protocol, and shipped on ice by overnight courier to the cell culture facilities. Isolation of canine MSCs was achieved by centrifuging whole marrow aspirates over a Percoll cushion, using procedures analogous to those developed for human MSC isolation (54). Tissue culture flasks (185 cm²) were seeded with 10' nucleated cells isolated from the cushion, and cultured with DMEM containing 10% fetal calf serum from a selected lot (80). Cells were passaged at 8 x 10³ cells/cm², and transported back to the veterinary hospital where they were maintained until the time of implantation. Cell-loaded implants were prepared by incubating fibronectin-coated porous hydroxyapatitetricalcium phosphate (HA/TCP) cylinders (Zimmer, Inc.) in a 7.5 x 10⁶ cells/ml suspension of MSCs for 3 hr at 37°C. The interval between marrow harvest and implantation was 16 days. An aliquot of cells from each preparation was also cultured under osteoinductive conditions to quantify aspects of osteoblastic differentiation.

### Canine Femoral Gap Model

A unilateral segmental femoral defect model was developed for this study following IACUC approval. Under general anesthesia, thirty-six skeletally mature female purpose-bred hounds (20 kg) underwent resection of a 21 mm long osteoperiosteal segment from their mid-diaphysis. A 4.5 mm Synthes* 8-hole lengthening plate was contoured to the lateral aspect of the bone, and secured with bicortical screws. The defect was filled with one of three materials; 1) a cell-free HA/TCP cylinder, 2) an MSC-loaded HA/TCP cylinder, or 3) cancellous bone harvested from the iliac crest. HA/TCP implants were secured by placing two sutures around the implant and the plate. Animals received peri-operative antibiotics, and analgesics were administered for three days post-operatively.

### Radiographic and Histologic Analyses

Standard radiographic images were obtained at pre-op, immediately post-op, and at 4 week intervals until termination of the study. All samples contained a radiodensity step wedge to provide a basis for comparing changes over time, and between dogs. Upon sacrifice, specimens were subjected to high resolution Faxitron radiography, and subsequently processed for biomechanical evaluation. Following torsion testing, undecalcified longitudinal sections will be processed for quantitative histomorphometry.

### Biomechanical Testing

Sixteen weeks after implantation, animals were sacrificed for torsion testing of femurs. The fixation plate, screws, and adherent soft tissue were removed, and the metaphyses of the bones were embedded. The specimens will be externally rotated in a custom torsion test apparatus, failure load and stiffness recorded, and the data analyzed by one way ANOVA according to *post hoc* Student-Newman-Keuls tests.

### Results

All animals tolerated the surgical procedure well, with no incidence of infection, implant rejection, or failure of fixation. Two modes of repair were apparent in the MSC-loaded samples; first, considerable callus formation occurred at both host-implant interfaces; and second, a substantial collar of bone surrounding the implant itself developed. Cell-free implants did not possess either of these features. Autograft samples underwent a traditional consolidation sequence, with the majority of bone laid down in the medial aspect of the gap defect. MSC-loaded samples not only became fully integrated at the host implant interface, but the periosteal collar extended proximally and distally beyond the cut edges of the gap. Furthermore, the diameter of new bone at the mid-diaphysis was greater in MSC-loaded implants than either autograft samples or intact limbs. Biomechanical analysis of harvested samples is currently in progress. *in vitro* analyses of the osteogenic potential MSCs from each animal demonstrate the development of alkaline phosphatase-positive cells which deposit significant mineralized extracellular matrix.

Preliminary histomorphometrical data from MSC-loaded (n=2) and cell-free (n=1) HA/TCP carrier shows that bone fill as percentage of available space is 39% and 7% respectively. In the case of the MSC-loaded samples, in addition to the considerable amount of bone in the confines of the ceramic block, there was also a fairly large mineralized periosteal callus. Also, the marrow space was reestablished within the defect. Whereas in the cell-free HA/TCP cylinders, most of the bone present was in the endosteal space with some penetration into the implant.

Torsional testing of the samples (n=6 per group) showed that the MSC-loaded samples were almost twice as strong as the cell-free samples, but were only a third as strong as autograft controls.

### Discussion

The present study demonstrates that MSCs from a large animal may be culture-expanded, and implanted for the successful repair of large diaphyseal bone defects. Radiographic and histologic evidence indicates that not only do the MSCs form bone within and around the implant directly, but their presence elicits a response in the host periosteum to form additional bone. The mechanism of this is currently not known, but is consistent with our observation that MSCs undergoing osteogenic differentiation secrete a paracrine factor(s) which is osteoinductive (63). The conspicuous lack of callus formation and periosteal reaction in the cell-free implants was an unexpected finding. Radiographic evidence suggests that MSC-mediated bone regeneration is faster than autograft throughout the study period. In addition to establishing a new standardized model for large animal bone repair, this study illustrates the feasibility of translating autologous stem cell therapy from the laboratory into the clinic.

### Example 3

### In vivo Bone Formation Using Human Mesenchymal Stem Cells

Although rat MSCs have been shown to synthesize structurally competent bone in an orthotopic site (68), human MSCs have only been shown to form bone *in vitro* (12,64) and in an ectopic implantation site in immunodeficient mice (55). Since fracture healing and bone repair depend on the ability to amass enough cells at the defect site to form a repair blastema, one therapeutic strategy is to directly administer the precursor cells to the site in need of repair. This approach is particularly attractive for patients who have fractures which are difficult to heal, or patients who have a decline in their MSC repository as a result of age (72,118), osteoporosis (128), or other metabolic derangement. With this in mind, the goal of the current study was to show that purified, culture-expanded human MSCs are capable of regenerating bone at the site of a clinically significant defect.

### Materials and Methods

### Human MSC Cultivation and Manipulation

Isolation and culture-expansion of human MSCs from a bone marrow aspirate obtained from a normal volunteer after informed consent was conducted as previously described (54,52). Following initial plating in Dulbecco's Modified Eagle's Medium (Sigma) containing 10% fetal bovine serum (BioCell) from a selected lot (80), non-adherent cells were removed on day 3 at the time of the first medium change, and fresh medium was replaced twice weekly thereafter. Adherent MSCs represent approximately 1 in 10⁵ nucleated cells originally plated. When culture dishes became near-confluent, cells were detached and serially subcultured.

### In Vitro Osteogenic Assays

Human MSCs were replated into six-well dishes at a density of 3x10³ cells/cm². The following day (Day 0), fresh medium was provided, and the cells were grown in the absence or presence of Osteogenic Supplements (OS) (12,64), Media changes were performed twice weekly, and at days 4, 8, 12 and 16, cultures were assayed for cell number, alkaline phosphatase (APase) biochemistry and histochemistry, and mineralized matrix production utilizing techniques previously described (64).

### Implant Preparation

Porous hydroxyapatite/β-tricalcium phosphate (HA/TCP) ceramic blocks, mean pore size 200-450 µm (Zimmer, Inc., Warsaw, IN), were shaped into cylinders approximately 4 mm in diameter and 8 mm in length with a 1 mm central canal, or cut into cubes 3 mm per side. MSC-loaded implants were prepared by incubating human fibronectin-coated HA/TCP cubes and cylinders in a 7.5 x 10⁶ cell/ml suspension of first passage MSCs for 2 hr at 37°C as previously described (68). Cell-free control cylinders were prepared identically.

### Athymic Rat Femoral Gap Model

The femoral gap surgical model employed here has been used extensively in euthymic rats to study long bone repair (68,129,34,147). Briefly, both femurs of Harlan Nude (Hsd:Rh-*rnu*) rats (325 g) were exposed by an anterolateral approach. A polyethylene fixation plate was attached to each femur by four Kirschner wires, and an 8 mm transverse segment of the central diaphysis, along with its adherent periosteum, was removed by using a rotary osteotomy burr under saline irrigation. Each animal then received a cell-free HA/TCP cylinder in one femoral defect, an identical cylinder loaded with human MSCs in the contralateral defect, and a subcutaneous implant of a MSC-loaded HA/TCP cube along the dorsum.

### Radiography

Immediately after sacrifice at each time point, all specimens were radiographed in a lateral position using a high resolution Faxitron Imaging system with an exposure of 35 kVP for 30 sec.

### Quantitative Histomorphometry and Immunochemistry

Upon sacrifice at 4, 8, and 12 weeks, a minimum of 3 specimens of each type were processed for undecalcified histology following radiography. Longitudinal sections were cut, stained with Toluidine blue-O, and quantitative assessment of bone formation was performed using Leica Quantimet 500MC image analysis software as previously described (68). The data were analyzed by Student's t-test. Subcutaneously implanted samples were fixed in formalin, decalcified, embedded in paraffin, serially sectioned, and similarly stained. Limbs from one animal at each time point were also prepared for immunostaining by monoclonal antibody 6E2, which distinguishes human cells from rat cells (54). Undecalcified cryosections were incubated with 6E2 supernatant, or an irrelevant primary monoclonal antibody control (SB-1) (10), followed by FITC-conjugated goat anti-mouse IgG secondary antibody (GIBCO) diluted 1:500 in phosphate-buffered saline.

### Biomechanical Testing

Twelve weeks after implantation, 7 experimental animals and 6 unoperated control animals were sacrificed for torsion testing of femurs as previously described (81). The fixation plate and adherent soft tissue were removed, and the metaphyses of the bones were embedded. The specimens were externally rotated in a custom torsion test apparatus, failure load and stiffness recorded, and the data analyzed by one way ANOVA with *post hoc* Student-Newman-Keuls tests.

### Results

### MSC Cultivation and Osteogenic Differentiation In Vitro

Human MSC cultures were established and, by 7 days, formed characteristic colonies on the surface of the culture dish. Primary colonies which were subcultivated on day 14 attached uniformly to the surface of new dishes, and were allowed to divide for another 7 days until they became ∼ 85 % confluent. Passaged cells demonstrated their characteristic spindle-shaped morphology (Fig. 6A), and uniformly divided resulting in an even distribution of MSCs throughout the plate. Cells derived from this first passage were used for preparing implants as previously described, and an aliquot was used to confirm their osteogenic potential *in vitro.*

As described in previous studies (12,64,13), MSCs cultured with OS underwent a dramatic change in cellular morphology from that of spindle-shaped to cuboidal, which was accompanied by an increase in APase activity and production of an extracellular matrix rich in bone hydroxyapatite (Fig. 6B). A significant increase in APase activity was observed after 4 days of OS treatment with maximal activity occurring on day 8, followed by a decline through day 16 (Fig. 6C). This late decrease in APase activity of OS cultures correlates with increasing mineral deposition and terminal differentiation of cells into osteocytes. While no calcium deposition was detected either by Von Kossa staining or the sensitive colorimetric quantitative calcium assay in Control cultures, Figure 6C illustrates that MSCs grown with OS deposited a significant amount of calcium by days 12 (60 ± 5.1 µg/dish) and 16 (98 ± 5.0 µg/dish).

### MSC-Mediated Osteogenesis in Ectopic HA/TCP Implants

Human MSC-loaded HA/TCP cubes implanted in the subcutaneous space of athymic rats displayed evidence of osteogenesis by 4 weeks, but considerably more bone was present within the pores at 8 and 12 weeks. A representative section from a MSC-loaded cube harvested 12 weeks following implantation is shown in Figure 7. Bone formation occurs within the pores of the cubes, and is associated with vascular elements which penetrate the implant. Such angiogenesis is obligatory to new bone formation since the secretory activity of osteoblasts is an oriented phenomenon guided by vasculature (25). As previously demonstrated (32,54), cubes implanted without MSCs never contained bone but were filled with fibrous tissue and blood vessels only.

### Osteotomy Model and Radiography

Figure 8A illustrates the segmental defect model used in this study. The polyethylene fixation plate on top of the femur provides stability following creation of the 8 mm diaphyseal defect. No animals experienced failure of fixation or other post-operative complications throughout the course of study. Previous studies have established that femoral defects that are not implanted with a bioactive material give rise to a fibrous non-union devoid of bone (68,34,147). High resolution Faxitron radiographs provided sufficient clarity and detail to discern subtle changes occurring within the implant and the surrounding host bone. Representative radiographs of the femurs from the 2 groups recovered 12 weeks post-implantation demonstrate substantially more bone in animals which received MSC-loaded HA/TCP cylinders (Fig. 8B) versus cell-free cylinders (Fig. 8C). Increasing radiodensity, and obliteration of the apparent pore structure, was used as an indication of new bone formation within the core of the implant. Although integration of the implant, or union, was not generally observed by 4 weeks, the subsequent formation of a radiodense bone bridge between the implant and the host at 8 weeks completely masked the interface. By 8 weeks, the MSC-loaded implant contained considerable bone within the pores and was integrated with the host bone at the ends of the implant. At 12 weeks, union was complete and additional bone was evident in the pores. Callus formation along the fixation plate was observed in some samples, as was an occasional eccentric spicule of bone usually present along the medial aspect of the femur. Some specimens, both with and without cells, contained cracks within the core of the implant.

### Immunocytochemical Evaluation

Immunocytochemical staining with antibody 6E2 demonstrates that, at 4 weeks, virtually all the cells within the pores of the implant were reactive on their surface and were, therefore, of human origin (Fig. 9A). Along the immediate periphery of the implant, the host rat cells were intermingled with the human donor cells, but as the distance away from the surface of the implant increased, the representation of donor cells precipitously declined. The presence of these peripheral cells which are not immunostained also serves as a negative control for this established antibody. The ceramic material itself, which appears black in the phase contrast micrograph (Fig. 9B), displays a high level of background fluorescence. The exquisite sensitivity of the 6E2 antigen:antibody interaction necessitated that we use unfixed frozen sections which, unfortunately, limited our ability to process these calcified tissue specimens for immunostaining. While we were able to obtain satisfactory cryosections of 4 week samples (shown here), we were unable to prepare sections from later samples which contained substantially more bone.

### Histologic Evaluation

Analysis of the Toluidine blue-O-stained samples confirmed the observations made by radiography. Photomicrographs of representative sections of the implant groups recovered at 12 weeks are shown in Figure 9. Most of the pores of the implants loaded with MSCs contained substantial new bone by 8 weeks, and this process of bone regeneration continued through the 12 week assessment period (Fig. 9C). At 8 weeks nearly all pores contained new bone, except in some discrete areas where loading of the MSCs may have been compromised. Evaluation of limbs following biomechanical testing indicates that fractures were of a transverse or spiral nature, and were generally propagated through a central region of the implant containing cartilage or a modest amount of bone, as seen in Figure 9C. During the regenerative process, substantial new bone formation occurred at the interface between the host and the implant, leading to a continuous span of bone across the defect. New woven and lamellar bone can be seen in intimate contact with the cut edge of the host cortex at 12 weeks (Fig. 9E), and this region of union is directly contiguous with bone formed throughout the pores of implant. In regions deeper within the HA/TCP (Fig. 9F), filling of the pores with new bone and vasculature is evident.

In defects fitted with the HA/TCP carrier alone, the pores of the implant were predominantly filled with fibrous tissue even at 12 weeks (Fig. 9D). Many samples had evidence of modest integration at the host-implant interfaces, and at one end of this representative implant (Fig. 9D), host-derived endosteal bone appears to be advancing into the medullary canal of the carrier as a result of osteoconduction. None of the cell-free ceramic carriers contained bone throughout the pores of the implant.

### Histomorphometric Evaluation

The results qualitatively described above are mirrored in the histomorphometric data presented in Table 3.

**Table 3**

| **Bone Fill in HA/TCP Implants as a Percentage of Available Space** | | | |
|---|---|---|---|
| | **Four Weeks** | **Eight Weeks** | **Twelve Weeks** |
| Carrier Alone | 1.89 ± 1.00 | 11.47 ± 7.08 | 29.51 ± 8.93 |
| Carrier plus MSCs | 1.95 ± 1.92 | 26.46 ± 3.60* | 46.61 ± 14.83* |

**Table 3.** Longitudinal sections through the segmental defect of athymic rats implanted with ceramic carriers, with and without human MSCs, were evaluated histomorphometrically for bone content . The results represent the mean ± SD of 3 experimental limbs of each group at 4 and 8 weeks, and 8 limbs of each group at 12 weeks. **P*<0.05 compared to the carrier alone at each time point.

Bone present in the cell-free HA/TCP implants primarily represents the bony ingrowth from the cut ends of the host cortices. At 4 weeks and beyond, the MSC-loaded samples contained significantly more bone than the cell-free group, and the average bone fraction within the implant increased over time, reaching 26.5% and 46.6% by the 8 and 12 week time points, respectively. This increased bone fraction at 8 weeks is 2.3-fold higher than that measured in cell-free implants at the same time, and by 12 weeks, is over 23-fold higher than that observed in either condition at 4 weeks. The volume fraction of the HA/TCP carrier remained constant, and served as an internal control for histomorphometry.

### Mechanical Testing

Twelve experimental and 11 intact femora from age and weight-matched control animals were tested in torsion 12 weeks after implantation. Two experimental limbs were not tested because they were extremely fragile. Gross inspection of the healed defects revealed a distal varus rotation deformation in most specimens. Table 4 summarizes the mechanical testing results in terms of torsional strength, stiffness, and total energy absorbed.

**Table 4**

| **Mechanical Properties of Rat Femora 12 Weeks after Implantation** | | | |
|---|---|---|---|
| | **Intact Control** | **Carrier Alone** | **Carrier+MSCs** |
| Strength(N mm) | 409 ± 71 | 74 ± 63 | 159 ± 37 |
| Stiffness(N·mm/deg) | 39 ± 5.5 | 6.6 ± 4.2 | 16.2 ± 4.0 |
| Energy(N·mm x deg) | 2.6 ± 0.7 | 0.6 ± 0.4 | 1.3 ± 0.8 |

**Table 4.** Mechanical testing data on rat femur samples from unoperated age matched controls (Intact Control), or animals whose segmental defects were implanted with the HA/TCP carrier alone (Carrier alone) or the MSC-loaded HA/TCP (Carrier + MSCs). These results represent the mean ± SD of 6 limbs from each experimental implant group, and 11 limbs from control animals. Twelve weeks after implantation, each specimen was harvested, the ends of the bone were embedded, and the samples were tested in external rotation at 6 degrees/second along the longitudinal axis until failure. One-way ANOVA on each of the parameters showed a significant difference between the groups at *P*<0.0001. Furthermore, each of the groups were significantly different from the other for strength and stiffness (*P*<0.05), as determined by *post hoc* Student-Newman-Keuls tests.

These results demonstrate a 115%, 145% and 112 % increase in strength, stiffness and torsional energy absorbed, respectively, in MSC-loaded samples compared to cell-free carrier samples. All three groups were found to be statistically different from each other in failure torque and stiffness.

### Discussion

The results presented here demonstrate that purified, culture-expanded human MSCs are capable of healing a clinically significant bone defect in a well-established model for bone repair. While the osteogenic potential of human MSCs has been proven by neo-osteogenesis in subcutaneous implants (54), as well as in studies of isolated MSCs *in vitro* (12,64), this is the first demonstration that human MSCs can form bone at an orthotopic site in need of repair. The combination of MSCs with a porous HA/TCP carrier possesses regenerative potential which is histomorphometrically and biomechanically superior to the carrier alone. This investigation paves the way for the clinical application of autologous MSC-therapy for the treatment of orthopedic defects in man.

The progressive increase in radiodensity of the healing bone at 8 weeks parallels the histological observations of processed limbs. Immunocytochemistry proves that the cells associated with the ceramic at 4 weeks are of human origin, and that the cells surrounding the implant are from the host. At 8 weeks and beyond, bone is laid down by the donor MSCs and eventually resorbed and replaced by bone derived from host cells through the normal remodeling sequence (24,47). It is important to note that in the process of regenerating this osseous defect, bone formation occurs by a direct conversion of mesenchymal cells into osteoblasts rather than by an endochondral cascade. This observation is consistent with previous studies of osteogenesis in implants loaded with animal or human MSCs (32,70,54,68). As the regenerative process continues, the pores of the ceramic are filled with an increasing amount of bone, which is laid down upon the walls of the implant or existing bone, and oriented by the invading vasculature that provides a portal for the entry and establishment of new marrow islands containing hematopoietic elements and host-derived MSCs.

The rate of bone regeneration is lower than that observed in euthymic rats implanted with syngeneic MSCs (68), suggesting that immunocompromised rats are not the ideal hosts to assess the bone-forming potential of human MSCs. This may be due in part to the xenogeneic nature of the implant and the increased natural killer cell activity, which may be a compensatory mechanism for the animal to cope with its deficient T-cell-mediated immunity (123). Nevertheless, a significantly higher amount of bone was formed in the defect which received MSCs compared to those limbs receiving the carrier only. The extent of host-implant union was greater in the MSC-loaded implants, which likely reflects the combined contributions of implanted MSCs and host-derived cells.

The ability of human MSCs to regenerate bone in this experimental model compares favorably with other investigations testing implants such as demineralized bone matrix, bone marrow, purified or recombinant bone morphogenic proteins (BMP), allograft, ceramics, fibermetals and gene-activated matrices (129,34,147). In addition to forming a substantial amount of histologically normal bone, the biomechanical data demonstrate that torsional strength and stiffness at 12 weeks were ∼ 40% that of intact control limbs, which is more than twice that observed with the cell-free carrier, and also twice that achieved in a similar study of bone repair using fresh autograft in a primate long bone defect model (29).

Recently, growth factors such as recombinant human BMP have been implanted in experimental bone defect models in an effort to stimulate bone repair (147,78,29). Although recombinant BMPs are capable of inducing the endochondral cascade in ectopic implants (146), their ability to reproducibly direct bone formation at orthotopic sites has been hampered by the problems associated with the design and selection of an appropriate carrier. In contrast to the mechanical data showing significant bone regeneration in a MSC-loaded ceramic, BMP delivered in the same HA/TCP carrier did not increase implant strength over the carrier alone (126). The brittle nature of this ceramic, combined with its slow resorption and complex porous structure, may explain why even in the presence of significant bone formation mechanical strength remains less than intact limbs. In addition, stress shielding of the new bone, as a result of the load-bearing fixation plate, also restricts the strength of the healing defect. We believe, as has been previously suggested (16), that the use of an osteosupportive HA/TCP cylinder may not be the ideal matrix for replacement of diaphyseal defects . Efforts at designing the optimal biomatrix carrier for the delivery of MSCs is an active area of investigation.

Implantation of culture-expanded autologous MSCs offers the advantage of directly delivering the cellular machinery responsible for synthesizing new bone, and circumventing the otherwise slow steps leading to bone repair. Even in patients with a reduced ability to regenerate connective tissue, presumably due to a low titer of endogenous MSCs (72,128,144,11), these rare MSCs may be isolated and culture-expanded over one billion-fold without a loss in their osteogenic potential (13), thus restoring or enhancing a patient's ability to heal tissue defects. The studies presented here suggest that MSC-based cell therapies will be useful for the reconstruction of a variety of tissue defects in man.

### Example 4

### Effect of Coating on the Osteogenic Response of MSC-Loaded HA/TCP Cubes

This experiment was performed in an attempt to establish that uncoated HA/TCP cubes are equivalent to fibronectin- or autologous serum-coated HA/TCP cubes in supporting MSC-mediated osteogenesis.

### Materials & Methods

Standard HA/TCP cubes coated with either fibronectin, 1% autologous serum, 10% autologous serum or those left uncoated, were loaded with MSCs and implanted subcutaneously into athymic mice. The cubes were retrieved six weeks post-implantation and inspected for the level of osteogenesis by decalcified histological methods. The experiments were done with multiple human and canine donors, and were performed in duplicate mice.

### Results & Conclusion

MSC-loaded cubes from all treatment groups showed a significant amount of bone formation at six weeks. The coating of HA/TCP cubes with either fibronectin or serum had no effect on the level of MSC-mediated osteogenesis within the cube. As expected, the cell-free control HA/TCP cubes did not have osteogenesis. Based on the above results, we conclude that uncoated HA/TCP is a viable carrier for the delivery of MSCs to effect bone repair/augmentation.

### Example 5

### The Comparison Of Bone Grafting Materials

### Using a Canine Spinal Fusion Model

The data reported here provides important information regarding the value of fresh bone marrow and of culture expanded purified mesenchymal stem cells as a means of improving existing graft materials for spinal fusion applications. This technology may result in a significant improvement in the efficacy of bone grafting materials and procedures. Furthermore, use of bone grafting procedures which do not require harvest of autogenous bone graft will significantly reduce the morbidity of bone grafting procedures.

### Materials & Methods

We developed the canine posterior segmental spinal fusion model specifically for evaluation and comparison of developmental bone grafting materials which had proven efficacy in small animals. Canine bone is a better model for human bone than rodents (35), and avoids many of the limitations associated with defect models discussed herein. Use of a larger graft volume and a common site for clinical graft procedures (the spine) are significant advantages. Testing three materials in each animal allows control for interanimal variation and reduces the number of animals required. The model allows each fusion site to be mechanically tested without artifacts from internal fixation. Union score of the cross sectional area of the fusion mass at the site of failure has also proven to be a sensitive means for comparison between materials. Quantitative CT image analysis of each fusion site prior to destructive testing was developed in response to the first review. This now allows quantitative analysis of fusion mass volume, mean electron density (mineralization), and cross sectional area.

Using this model, we have shown that a collagen/ceramic (CC) composite composed of Type I bovine fibrillar skin collagen and 0.5-1.0 mm diameter granules of a biphasic calcium phosphate ceramic (60% hydroxyapatite, 40% tri-calcium phosphate), is ineffective as a graft material. Used alone it is no better than an ungrafted defect. Addition of an extract of bone matrix proteins or adding autogenous bone improved efficacy (p<0.01), but the result remained inferior to pure autogenous bone (p < 0.01)(55). We also showed that adding this collagen/ceramic composite to autogenous bone as a "bone graft expander" significantly reduced autograft performance (p<0.01)(58). Interestingly, successful fusions resulting from the CC composite had similar mechanical properties to fusions resulting from autograft, indicating that residual unresorbed ceramic granules had no evident adverse effect on the material properties of the fusion mass.

We have evaluated bone marrow processing as a means of enhancing graft performance in a spinal fusion study, using the Collagen Corporation collagen/ceramic composite as a delivery system for a purified matrix protein, Osteoinductive Factor (OIF). This matrix was mixed 50:50 with either autogenous cancellous bone (AB), freshly aspirated bone marrow (ABM), or fresh bone marrow nucleated cells which had been concentrated ten fold by centrifugation and buffy coat isolation (BMC). Unfortunately, as the study was completed, it was discovered that OIF was not an active cytokine. Earlier preparations of OIF had been inductive due to contamination with BMPs. As a result, the overall fusion rate was low. However, there was a strong trend for improved results from marrow concentration when ABM and BMC are compared (p=0.06, Logistic Regression Model for Clustered Data). Unfortunately, our ability to answer this important question was limited by the poor baseline performance of the matrix. This highlights the importance of selecting an effective and reliable matrix for evaluation of bone marrow grafts in this project. The incidence of union scores from 0 to 4 for each material in this experiment are presented in Table 5.

**Table 5**

| **Spinal Fusion Union Score for Selected Implant Materials** | | | | | | | |
|---|---|---|---|---|---|---|---|
| | **Union Score** | | | | | | **Total Score** |
| **Materials** | N | 4 | 3 | 2 | 1 | 0 | |
| AB+OIF/CC | 11 | 2 | 1 | 1 | 2 | 5 | 15 |
| BMC+OIF/CC | 11 | 0 | 2 | 2 | 1 | 6 | 11 |
| ABM+OIF/CC | 11 | 0 | 2 | 0 | 0 | 9 | 6 |

These studies show that the posterior segmental canine spinal fusion model is a sensitive and reliable tool for comparison of graft materials. Ungrafted sites heal poorly with a low union score. Autogenous cancellous bone graft is very effective, but does not heal every graft site. This spectrum of performance for autogenous bone makes it possible to evaluate materials which may perform as well as or better than autogenous cancellous bone. Both union score and mechanical testing can and have been used effectively to compare materials. The use of high resolution CT image analysis adds an important nondestructive assessment tool which is flexible, quantitative and reproducible. This model sets a new standard for the evaluation of bone grafting materials.

### Posterior Segmental Canine Spinal Fusion Model

The specific aims of this project address the comparison of three graft materials: Granular Ceramic matrix loaded with autogenous mesenchymal stem cells; Granular Ceramic matrix loaded with fresh bone marrow; and Granular Ceramic matrix loaded with fresh bone marrrow and autogenous mesenchymal stem cells.

Twelve male beagle dogs (age 10-14 months, 12-14 kg) are used for the experiment. Localized fusions are performed at three spinal fusion sites, L1-2, L3-4, and L5-6. Each site is internally fixed using dual plates immobilizing adjacent spinous processes, separated by one mobile segment. Each animal is grafted with one of the three materials under evaluation. To limit the potential for surgical bias and to insure distribution of materials at each of the three graft sites, twelve cards, two sets of the six possible combinations of the three materials in three sites, are prepared at the beginning of each experiment and placed in an envelope. Site assignments are then made intraoperatively by blind drawing after site preparation is complete. Internal fixation is applied to each segment using plates placed on either side of the spinous processes. No external immobilization is used. All animals are euthanized twelve weeks post-operatively lateral faxitron radiographs of each excised spine are obtained to assess the integrity of fixation. After removal of plates, high resolution CT images are acquired of all segments from L1-L6 in each spine. Individual fusion segments are mechanically tested to failure and physically evaluated for union. Comparison between materials is made based on union score, quantitative image analysis of CT data, and the mechanical properties observed for each fusion.

Three weeks prior to spinal fusion each animal undergoes aspiration of bone marrow from the left iliac crest using sterile techniques under short acting IV sedation. These samples will then be transported to isolate and proliferate mesenchymal stem cells.

On the day of surgery, each animal in the study undergoes spinal fusion at three separate levels. Mesenchymal stem cells are provided, and fresh bone marrow will be harvested again, this time from the right iliac crest. Each material composite is then prepared intraoperatively. Following preparation of the surgical bed and application of internal fixation, a 2 cc volume of each material is grafted at one level in each animal. In this way each animal has one fusion site for each of the materials. The materials are distributed according to a randomization protocol to prevent surgical bias and insure uniform distribution of materials by site.

### Initial Bone Marrow Aspiration and MSC Preparation

Following sterile skin preparation, a small (3 mm) stab incision is made using a #11 blade. A Lee-Lok bone marrow aspiration needle (Lee-Lok Inc., Minneapolis, MN), is advanced into the bone cavity. The obturator is removed. A 2 cc volume of bone marrow is then aspirated promptly into a 10 cc syringe containing 1 cc of heparinized saline-(1000 units/ml). The syringe is detached and inverted several times to insure-mixing. Subsequent aspirates are taken using identical technique through the same skin incision and the same cortical window, but redirecting the needle tip to intramedullary sites separated by at least 1 cm. Pressure is held on the site for 3-5 minutes to insure hemostasis. No dressing is necessary. No post-operative immobilization or restriction is required.

### MSC Isolation and Cultivation

Iliac crest marrow aspirates (10 ml) were obtained from 15-25 kg random source canines under Propofol anesthesia (Diprivan 1%, Stuart Pharm., DE). The marrow was drawn into a 10 ml syringe containing 7500 units of Heparin (Elkins-Simm, Cherry Hill, NJ) to prevent clotting. For some canines, the marrow was placed on ice and shipped overnight to the cell culture facility. The marrow was mixed with two volumes of Complete media, consisting of 10% FBS and antibiotics (100 U/ml penicillin G, 100 µg/ml streptomycin sulfate, and 0.25 µg/ml amphotericin B) in low-glucose DMEM. The nucleated cell fraction of the marrow was enriched for MSCs based on density separation on a 1.063 g/cc Percoll cushion. Two hundred million nucleated cells in 5 ml of media were carefully layered over 20 ml of Percoll (Sigma, St. Louis, MO). Separation was achieved by centrifugation at 400 x g for 20 minutes. Cells collecting at the media-Percoll interface were then washed and plated in 100 mm petri-dishes at 1.6 x 10⁴ cells/cm² in 7 ml of Complete media, or in T-185 culture flasks at 5.4 x 10⁴ cells/cm² in 32 ml of Complete media. The cells were incubated at 37 °C in a humidified 5% CO₂ environment. On day 4 of culture, the non-adherent cells were removed along with the culture media. The cultures were fed twice weekly and passaged between days 10-13 by releasing the cells with 0.05% Trypsin 0.53 mM EDTA exposure for 5 minutes. The cells were replated at 8 x 10³ cells/cm² for all subsequent passages. For some experiments, additional passaging was performed when cells approached confluence, typically 5-7 days after plating.

### Spinal Fusion Surgical Procedure

Under general endotracheal anesthesia a mid-line posterior longitudinal incision is made from T10 to L7. Cutting cautery is used to outline the tips of spinous processes L1 through L6 and subperiosteal elevation of paraspinal muscles is performed. The interspinous ligament and interlaminar tissue are excised at the L1-2, L3-4, and L5-6 interspaces preserving the ligamentum flavum. A dental burr is used to excise facet cartilage to the level of subchondral bone and to perform a superficial decortication of the adjoining laminar surfaces under continuous saline irrigation. Entry into the neural canal is avoided. When site preparation is complete, a saline soaked gauze is placed in each fusion site, the wound is covered; and an unscrubbed assistant blindly selects a card containing the material/site assignment for that animal.

Porous hydroxyapatite/tricalcium phosphate (HA/TCP) ceramic material, supplied from Zimmer Inc. as a 60/40 combination, is lightly crushed and sieved to select for a particle size ranging from 1.0 to 2.5 mm in diameter. Following sterilization of the granules, 15 million MSCs are incubated with the 1 cc of ceramic for 3 hours at 37 degrees Celsius with agitation every 30 minutes. As noted above, implant groups consist of 1) ceramic granules combined with autologous MSCs, 2) ceramic granules combined with autologous MSCs and fresh bone marrow obtained by aspiration, and 3) ceramic granules combined with fresh bone marrow alone.

Following preparation of all graft materials, materials are carefully placed in their appropriate sites. Approximately 1 cc of each graft is used to fill the area of the excised facets and the interlaminar space. The remainder of each graft is layered over the dorsal surface of the adjoining lamina.

Fixation at each site is then applied. A 1 mm burr is used to place a hole in the central region of the distal spinous process at each site. 316L stainless steel plates (0.125" x 0.4" x 1.4") are placed on either side of the adjoining spinous processes and fixed to the caudal spinous process using a stainless steel bolt and nut (size 2-56, 0.5" long).
Four symmetrical holes in each plate allow selection among three potential fixation lengths to accommodate variation in the interspinous distance at individual sites (0.75", 0.90", and 1.05"). The fixation to the cranial spinous process at each level is accomplished by drilling through the appropriate hole in the plates to create a hole in the spinous process and by passing a second bolt and nut. Both bolts are tightened firmly without crushing the spinous processes. A second locking nut is then applied at each site to prevent loosening. The spinal wound and the autograft donor site is then closed using O-Dexon Plus sutures in deep fascia, interrupted subcutaneous sutures of 2-0 Dexon Plus, followed by staples in the skin.

Following sterile skin preparation, a small (3 mm) stab incision is made using a #11 blade. A Lee-Lok bone marrow aspiration needle (Lee-Lok Inc., Minneapolis, MN), is advanced into the bone cavity. The obturator is removed. Bone marrow is then aspirated promptly into a 10 cc syringe, and mixed with the granular ceramic matrix and allowed to set for 10-15 minutes to allow clot formation. Excess fluid is then pressed out with a gauze sponge and a 2 cc volume of the matrix impregnated with fresh bone marrow is implanted.

### Animal Care

Study animals are cared for in accordance with the Principles of Laboratory Care and The Guide for the Care and Use of Laboratory Animals, NIH Publication No. 85-23, 1985.

Each animal receives prophylactic antibiotics of Penicillin G 500,000 units IM preoperatively and Ampicillin 250 mg po per day for five days post-operatively.
Acepromazine and Tylenol are used for perioperative pain. No external immobilization is used. Animals are housed in cages for three days post-operatively and then moved to runs where they are exercised daily.

### Medications

| | |
|---|---|
| Premedication: | - Atropine (0.02 mg/lb) [M |
| | - Penicillin G 1.2 million U IM |
| Sedation for 1st Aspirate | - Pentobarbital 20-25mg /kg IV |
| Induction | - Na Thiamylal (Surital) 6-8 mg/lb IV |
| Anesthesia | - Closed hallothane and oxygen |
| Post-op | - Penicillin G 250 mg IM qd x 5d |
| Distress/pain | - Acepromazine 2.2 mg/10 kg IV pm |
| Euthanasia | - Pentobarbital 50 mg IVP |

### Specimen Harvest

After twelve weeks animals are euthanized by pentobarbital overdose and the lumbar spine is harvested intact. Lateral Faxitron radiographs are obtained to document the integrity of fixation. After removal of the plates individual segments are meticulously cleaned of soft tissue, taking care not to damage the fusion mass and to prevent dehydration of the specimen. Segments are then potted in Orthodontic Resin (Meer Dental Supply, Cleveland, OH) and frozen in double sealed plastic bags at -20°C.

### Quantitative CT Image Analysis of Fusion Sites

Quantitative assessment of the fusion mass is performed using helical X-ray Computed Tomography (CT) and automated 3-dimensional image processing techniques. Quantitative measures at each fusion site includes: 1) fusion mass volume, 2) area measured at the mid-disk cross-sectional plane, and 3) mineralization density of the fusion mass.

Scanning is done on a Somatome Plus 40 CT scanner (Siemens Medical Systems). The specimen is placed with the cranial-caudal axis perpendicular to the direction of table travel. In this orientation the intervertebral spaces are oriented perpendicular to the scan plane. This reduces the chance of missing features of nonunions occurring in the transaxial plane and allows the midline of the intervertebral space to be identified more accurately. Scans will be done at 120 kVp, 210 mA, 1 sec helical mode, 2 mm collimation, and table speed 2 mm/sec, for 30 secs. This will produce images with 2 mm section thickness and 1 mm² pixel area. Images are then reconstructed using the bone algorithm and an image-to-image overlap of 1 mm. This provides the highest possible spatial resolution available in three dimensions (i.e. a voxel size of 1 mm³). A Siemens bone mineral density phantom is placed beneath each specimen to provide a reference for the quantitation of bone mineralization density.

Bone and soft tissue CT values are easily differentiable (CT bone - 150-1000, CT soft tissue - 0∼20). Therefore, three-dimensional segmentation of the reconstructed volume data sets is performed using a basic automated threshold algorithm in each two dimensional slice followed by a connectivity algorithm between slices. Once the three-dimensional data set has been segmented, the volume of the fusion mass is measured in a specified region of interest, defined by anatomic fiducial markers. The user selects two points which define the dorsal most points of the left and right neural foramen at the level of interest and one level above and below the site of interest. The user also identifies the midpoint of the intervertebral disk. The interactive tools for manipulating and visualizing the 3-D data sets are developed using the GL library and Iris Inventor Toolkit available on a Silicon graphics workstation.

The volume of the fusion mass is calculated by summing the segmented voxels (bone) within the specified region of interest and multiplying this sum by the appropriate voxel volume (1 mm³). The area of the fusion mass will be measured at the mid-fusion cross-sectional plane by summing the number of segmented pixels (bone) in the mid-fusion slice and multiplying the sum by the appropriate pixel area *(1 mm²).* A mean-mineralization density is calculated for the entire fusion mass and referenced to the density of the phantom. A 2-D topographic map of bone mineral density at the mid-disk cross-sectional plane is provided. The original volume of bone and bony cross section from the region of interest for each segment can be estimated by measuring the same region in the adjacent normal segment(s). This is used to normalize data from each fusion segment.

Software for this quantitative analysis requires four modules: 3-D segmentation, animation to view cross-sectional planes and mark fiducial points, calculation of rectangular region of interest and quantitative measures of bone fusion, and an interactive display module to view reconstructed data in 3-D.

### Mechanical Testing

Prior to testing, each specimen is thawed for 24 hours at room temperature. Testing is performed on an MTS TESTAR Bionix System using a custom four point bending device. After three sinusoidal conditioning cycles, load displacement data are collected nondestructively in flexion-extension and left-right bending. Failure testing is then performed in right bending using the ramp function at 8 mm/sec. Bending has been selected as the mode of failure because bending stiffness was found to be most closely correlated with union status in prior studies (94). Load-displacement curves are used to derive stiffness, maximum load, displacement to failure, and total energy to failure.

### Union Score

Immediately after mechanical testing, the surfaces of the fractured specimen are examined using a metal probe. By comparing both sides of the fracture surface, the degree of union is scored from zero to four based on a regional grid system (Figure 11). A score of four is defined as complete fusion of both facet joints and the entire lamina. One half point is added for union in either half of each facet joint or any of four quadrants of the adjoining laminar surfaces. Therefore, scores of 0, 1, 2, 3 and 4 represent union of roughly 0, 25, 50, 75 and 100 percent of the cross-sectional area of the grafted volume, respectively. Scoring is done by consensus of two observers who examine the specimens,together and are blinded with respect to the material grafted at each site.

### Statistical Analysis of Spinal Fusion Union Score and CT Data

Union score and CT generated data are analyzed to determine whether outcome differs statistically due to site and/or material effects. The data set is considered "incomplete" since only one material can be tested at each site within an animal. Therefore, both site and material are repeated factors but with missing observations. To accommodate the missing data profile of the experimental design as well as the possible correlation of observations within the same animal, a regression model for repeated measures data is used. This modeling strategy uses the Generalized Estimating Equation (GEE) approach described by Zeger *et al*. (150) and Ou *et al*. (108). Since union scores for a given dog are potentially not independent of each other, a working covariance structure of independence and a robust variance estimate are used in fitting the model. The covariance structure cannot be ignored without impacting inferences on the regression coefficients for site and material effects. T-tests using the robust variance estimates are used for specific comparisons between materials and sites as described by Paik (112). Union score remains the primary outcome parameter, however, distributional properties of the CT data will eventually dictate the statistical modeling strategy.

### Statistical Analysis of Spinal Fusion Mechanical Testing Data

Mechanical parameters are strongly influenced by union score. Therefore, mechanical testing is used in this model as a secondary outcome parameter, primarily as a means to compare the material properties of bone formed in complete fusions (Large cross sectional area-Union Score 3.5 to 4.0) induced by different materials. Selection of complete unions allows comparison of fusions having similar moments of inertia. The complexity of bony geometry in partial fusions and wide local variation in the quality of ununited soft tissue in the graft site prevents useful analysis of partial unions. Choice of this strategy reflects our practical belief that unless union score is high, the material properties of the bone formed by a given graft material will not be clinically important. The Student t-test has been used for first order comparisons. Paired analysis is most appropriate, but is not possible unless the union rates of the two materials to be compared are high.

### Interim Results of Spinal Fusion Study in Large Canines

When 15 million MSCs are incubated per 1 cc of granular ceramic, using the techniques for creating MSC-loaded ceramic granules described in this application, we have found that greater than 95 % of MSCs provided are adherent to the ceramic material after a 3 hour incubation period at 37 degrees Celsius. The MSC-ceramic material fits nicely into the surgical defect site created, without evidence of any migration of the implanted material up to 12 weeks post-operatively. Samples, either with or without MSCs, that were combined with fresh marrow at the time of implantation formed a soft clot which loosely covered the ceramic. No technical problems were encountered in the preparation or implantation of any samples. Analysis of the cells derived from each animal demonstrated osteogenic potential *in vitro* and significant bone formation in the standard *in vivo* ectopic implantation assay.

All animals were terminated 12 weeks postoperatively. Plain film radiographs demonstrate the presence of osseous union in nearly all animals receiving MSC-based implants. Three-dimensional reconstruction from 2mm thick CT scans obtained at 1 mm intervals through the surgical site illustrate a larger fusion mass in those regions containing implants with MSCs compared to implants containing fresh marrow alone. Fusion scores of samples containing MSCs were also higher than those containing only fresh marrow. Furthermore, these fusion scores were also higher than that achieved in a previous study using samples prepared in a similar fashion containing recombinant human BMP-2.

### Example 6

### Bone Defect Repair Using Bone Marrow

### in an Absorbable Collagen-Containing Sponge

The objectives of this study were to demonstrate efficacy of bone marrow and/or mesenchymal stem cells (MSCs) in healing clinically significant bone defects in an established animal model.

### Materials & Methods

In the study, Fisher 344 rats (Charles River Laboratories, Wilmington, MA) of approximately 325 grams in weight were used. A bilateral femoral gap 8 mm in length was created in each femur. This length is approximately towards the diameter of the mid-diaphysis of the femur. An internal fixation plate was applied with four Kirschner wires. The groups for comparison were separately treated with one of the following:
(1) Gelfoam^{®} sterile sponge (Upjohn - Kalamazoo, MI);
(2) Gelfoam^{®} sterile powder;
(3) Peripheral blood clot;
(4) Peripheral blood clot plus marrow derived from four bones;
(5) Gelfoam^{®} sponge containing marrow derived from four bones.
(6) Gelfoam^{®} sponge plus varying amounts of marrow from one bone down to one-half of one bone in the presence and absence of fresh peripheral blood to provide clot.

In this animal system, fresh marrow from four bones yields approximately 150 million cells while fresh marrow from one-half of one bone yields approximately 20 million nucleated cells. Each group consisted of a minimum of three animals, all of which were sacrificed six weeks post-operatively to obtain the desired end-points. Some animals received high-resolution Faxitron radiographs at an intermediate point three weeks after implantation. At the six-week time point when all animals were sacrificed, the limbs were removed, radiographed, and prepared for undecalcified histological evaluation.

Handling properties of the Gelfoam^{®} sponge, in combination with fresh marrow in the presence or absence of fresh peripheral blood clot, was desirable and nearly equivalent.

### Results

Evaluation of radiographs following sacrifice of the animals at 6 weeks revealed no bone in the defect region of those animals implanted with either Gelfoam^{®} sponge alone or those animals implanted with fresh marrow and a peripheral clot. Minimal endosteal spiking of new bone at the cut edges of the defect was observed, as is the case with the historical control of no implant alone. By contrast, those animals receiving Gelfoam^{®} sponge plus marrow from four bones or one bone, in the absence or presence of peripheral clot, demonstrated a robust osteogenic healing response in the region of the implant. Those animals implanted with Gelfoam^{®} sponge and marrow from one-half of one bone in the presence of peripheral clot demonstrated only modest amounts of bone formation. Finally, those animals implanted with Gelfoam^{®} sponge and marrow from one-half of one bone in the absence of fresh peripheral clot demonstrated no bone in the defect region. Histologic analysis of all of the specimens confirms the observations made based on high-resolution radiographs. The formation of neocortices in samples of Gelfoam^{®} sponge loaded with marrow cells was impressive. Histologic evaluation also indicates that no residual Gelfoam^{®} material was retained at the site of the implant six weeks following surgery. Samples of Gelfoam^{®} sponge loaded with marrow from one bone demonstrated islands of developing hemaetopoietic elements in the medullary canal. Host-implant interfaces appear to be intact.

In summary, significant osteogenic response of syngeneic marrow in each of the recipient rats which were implanted with Gelfoam^{®} sponge indicates the suitability of this cell and matrix combination implantation for the repair of significant bone defects.

### Cited Literature

1. Armitage JO, and Klassen LW. Bone Marrow transplantation, New York, - Churchill Livingstone, 1984: 1085-1111. (Koeple JA, ed. In: Laboratory Hematology)
2. Ashton BA, Abdullah F, Cave J, et al.: Characterization of cells with high alkaline phosphatase activity derived from human bone and marrow: preliminary assessment of their osteogenicity. Bone 6:313-9, 1985.
3. Aurori BF, Weierman RJ, Lowell HA, Nadel Cl, and Parsons JR.: Pseudarthrosis after spinal fusion for scoliosis. A comparison of autogeneic and allogeneic bone grafts. Clinical Orthopaedics & Related Research 199:153-158, 1985.
4. Bellows CG, Aubin JE, Heersche JN, and Antosz ME.: Mineralized bone nodules formed in vitro from enzymatically released rat calvaria cell populations. Calcified Tissue International 38:143-54, 1986.
5. Beresford, J.N. : Osteogenic stem cells and the stromal system of bone and marrow. Clin. Orthop. Rel. Res. 240:270-280, 1989.
6. Bolander ME, and Balian G.: The use of demineralized bone matrix in the repair of segmental defects. Augmentation with extracted matrix proteins and a comparison with autologous grafts. Journal of Bone & Joint Surgery American 68:1264-74, 1986.
7. Bos GD, Goldberg VM, Powell AE, Heiple KG, and Zika JM.: The effect of histocompatibility matching on canine frozen bone allografts. Journal of Bone & Joint Surgery American 65:89-96, 1983.
8. Bos GD, Goldberg VM, Zika JM, Heiple KG, and Powell AE.: Immune responses of rats to frozen bone allografts. Journal of Bone & Joint Surgery American 65:239-246, 1983.
9. Brandwein JM, Callum J, Rubinger M, Scott JG, and Keating A.: An evaluation of outpatient bone marrow harvesting [see comments]. Journal of Clinical Oncology 7:648-50, 1989.
10. Bruder, S.P., and Caplan, A.I. (1990) Bone 11, 133-139.
11. Bruder, S.P.; Fink, D.J.; and Caplan, A.I.: Mesenchymal stem cells in bone development, bone repair, and skeletal regeneration therapy. J. Cell. Biochem. 56:283-294, 1994.
12. Bruder, S.P.; Eames, B.F.; and Haynesworth, S.E.: Osteogenic induction of purified human mesenchymal stem cells in vitro: Quantitative assessment of the osteoblastic phenotype. Trans. Ortho. Res. Soc. 20:464, 1995
13. Bruder, S.P.; Jaiswal, N.; Haynesworth, S.E.: Growth kinetics, self-renewal and the osteogenic potential of purified human mesenchymal stem cells during extensive subcultivation and following cryopreservation, (1997) J. Cell Biochem. 64(2):278-294.
14. Bruder, S.P., Lawrence, E.G., and Haynesworth, S.E. (1995) Trans. Ortho. Res. Soc. 20, 8.
15. Bucholz, R.W., Carlton, A., and Holmes, R.E. (1987) Orthop. Clin. North Am. 18, 323-334.
16. Bucholz RW, Carlton A, and Holmes R.: Interporous hydroxyapatite as a bone graft substitute in tibial plateau fractures. Clinical Orthopaedics & Related Research 240:53-62, 1989.
17. Buckner CD, Clift RA, Sanders JE, et al.: Marrow harvesting from normal donors. Blood 64:630-4, 1984.
18. Burton CV, Kirkaldy WW, Yong HK, and Heithoff KB.: Causes of failure of surgery on the lumbar spine. Clinical Orthopaedics & Related Research 157:191-199, 1981.
19. Burwell RG.-. The function of bone marrow in the incorporation of a bone graft. [Review]. Clinical Orthopaedics & Related Research 200:125-141, 1985.
20. Burwell RG.: Studies in the transplantation of bone. 8. Treated composite homograft-autografts of cancellous bone: an analysis of inductive mechanisms in bone transplantation. Journal of Bone & Joint Surgery British 48:532-66, 1966.
21. Burwell RG.: Studies in the transplantation of bone.7. Journal of Bone & Joint Surgery British 46:110, 1964.
22. Caplan, A.I.: Mesenchymal stem cells. J. Orthop. Res. 9:641-650, 1991.
23. Caplan, A.I.; Fink, D.J.; Goto, T.; Linton, A.E.; Young, R.G.; Wakitani, S.; Goldberg, V.M.; and Haynesworth, S.E.: Mesenchymal stem cells and tissue repair. In The Anterior Cruciate Ligament: Current and Future Concepts. D.W. Jackson, ed. Raven Press, Ltd., New York. 405-417, 1993.
24. Caplan, A.I., and Bruder, S.P. (1997) in Textbook of Tissue Engineering, eds. Lanza, R., Langer, R., and Chick, W. (R.G. Landes Company, Georgetown), pp. 603-618.
25. Caplan, A.I. and Pechak, D. (1987) in Bone and Mineral Research/5, ed. Peck, W.A. (Elsevier, New York), pp. 117-183.
26. Connolly, J.F.; Guse, R.; Lippiello, J.; and Dehne, R.: Development of an osteogenic bone-marrow preparation. J. Bone Joint Surg. 71(5):684-691, 1989.
27. Connolly, J.F.; Guse, R.; Tiedeman, J.; and Dehne, R: Autologous marrow injection a a substitute for operative grafting of tibial nonunions. Clin. Orthop. Rel. Res. 266:259-270, 1991.
28. Cook SD, Reynolds MC, Whitecloud TS, et al.: Evaluation of hydroxylapatite graft materials in canine cervical spine fusions. Spine 11:305-9, 1986.
29. Cook, S.D., Wolfe, M.W., Salkeld, S.L., and Rueger, D.C. (1995) J. Bone Joint Surg. 77-A, 734-750.
30. Cornell CN, Lane JM, Chapman M, et al.: Multicenter trial of Collagraft as bone graft substitute. Journal of Orlhopaedic Trauma 5:1-8, 1991.
31. Dennis JE, and Caplan Al.: Porous ceramic vehicles for rat-marrow-derived (Rattus norvegicus) osteogenic cell delivery: effects of pretreatment with fibronectin or laminin. Journal of Oral Implantology 19:106-15, 1993.
32. Dennis, J.E.; Haynesworth, S.E.; Young, R.G.; and Caplan, A.I: Osteogenesis in marrow-derived mesenchymal cell porous ceramic composites transplanted subcutaneously: Effect of fibronectin and laminin on cell retention and rate of osteogenic expression. Cell Transplant 1:23-32, 1992.
33. Egrise, D.; Martin, D.; Vienne, A.; Neve, P.; and Schoutens, A.: The number of fibroblastic colonies formed from bone marrow is decreased and the in vitro proliferation rate of trabecular bone cells increased in aged rats. Bone 13:355-361, 1992.
34. Einhorn, T. A.; Lane, J. M.; Burstein, A. H.; Kopman, C. R.; and Vigorita, V. J.: The healing of segmental bone defects induced by demineralized bone matrix. J. Bone Joint Surg. 66(2):274-279, 1984.
35. Eitel F, Seller H, and Schweiberer L.-. [Morphological examination of animal-experiment results: comparison with regeneration of the human bone-structure. 11. Research results (author's transl)]. [German]. Unfallheilkunde 84:255-64, 1981.
36. Fang, J., Zhu, Y-Y., Smiley, E., Bonadio, J., Rouleau, J.P., Goldstein, S.A., McCauley, L.K., Davidson, B.L., and Roessler, B.J. (1996) Proc. Natl. Acad. Sci. USA 93, 5753-5758.
37. Feighan, J.E.; Davy, D.; Prewett, A.; and Stevenson, S: Induction of bone by a demineralized bone matrix gel: a study in a rat femoral defect model. J. Orthop. Res. 13:881-891, 1995.
38. Flatley TJ, Lynch KL, and Benson M.: Tissue response to implants of calcium phosphate ceramic in the rabbit spine. Clinical Orthopaedics & Related Research 179:246-52, 1983.
39. Friedenstein AJ.: Precursor cells of mechanocytes. [Review]. International Review of Cytology 47:327-59, 1976.
40. Friedenstein AJ, Chailakhyan RK, Latsinik NV, Panasyuk AF, and Keiliss Bi.: Stromal cells responsible for transferring the microenvironment of the hemopoietic tissues. Cloning in vitro and retransplantation in vivo. Transplantation 17:331-40, 1974.
41. Friedenstein AJ, Piatetzky SI, and Petrakova KV.: Osteogenesis in transplants of bone marrow cells. Journal of Embryology & Experimental Morphology 16:381-90, 1966.
42. Friedlaender GE, Strong DM, and Sell KW.: Studies on the antigenicity of bone. 11. Donor-specific anti-HLA antibodies in human recipients of freeze-dried allografts. Journal of Bone & Joint Surgery American 66:107-12, 1984.
43. Gepstein R, Weiss RE, and Hallel T.: Bridging large defects in bone by demineralized bone matrix in the form of a powder. A radiographic, histological, and radioisotope-uptake study in rats. Journal of Bone & Joint Surgery American 69:98492, 1987.
44. Gerhart, T.N.; Kirker-Head, K.; Kriz, M.J.; Holtrop, M.E.; Hennig, G.E.; Hipp, J.; Schelling, S.H.; and Wang, E.: Healing segmental femoral defects in sheep using recombinant human bone morphogenic protein. Clin. Orthop. Rel. Res. 293:317-326, 1993.
45. Glowacki, J.; Kaban, L.B.; Murray, J.E.; Folkman, J.; and Mulliken, J.B.: Application of the biological principle of induced osteogenesis for craniofacial defects. Lancet 1:959-968, 1981.
46. Glowacki J, and Multiken JB.: Demineralized bone implants. [Review]. Clinics in Plastic Surgery 12:233-41, 1985.
47. Goshima, J.; Goldberg, V.M.; and Caplan, A.I.; The origin of bone formed in composite grafts of porous calcium phosphate ceramic loaded with marrow cells. Clin. Orthop. Rel. Res. 269:274-283, 1991.
48. Grande, D.A., Southerland, S.S., Manji, R., Pate, D.W., Schwartz, S.E., and Lucas, P.A. (1995) Tissue Engin. 1(4), 345-353.
49. Grundel, R.E.; Chapman, M.W.; Yee, T.; and Moore, D.C.: Autogeneic bone marrow and porous biphasic calcium phosphate ceramic for segmental bone defects in the canine ulna. Clin. Orthop. Rel. Res. 266:244-258, 1991.
50. Harakas NK.: Demineralized bone-matrix-induced osteogenesis. [Review]. Clinical Orthopaedics & Related Research 188:239-251, 1984.
51. Haynesworth, S.E., Baber, M.A, and Caplan, A.I. (1995) Trans. Ortho. Res. Soc. 20, 7.
52. Haynesworth, S.E., Baber, M.A, and Caplan, A.I. (1996) J. Cell Physiol. 166(3), 585-592.
53. Haynesworth SE, Baber MA, and Caplan Al.: Cell surface antigens on human marrow-derived mesenchymal cells are detected by monocional antibodies. Bone 13:69-80, 1992.
54. Haynesworth, S.E.; Goshima, J.; Goldberg, V.M.; and Caplan, A.I.: Characterization of cells with osteogenic potential from human marrow. Bone. 13:81-88, 1992.
55. Healey JH, Zimmerman PA, McDonnell JM, and Lane JM.: Percutaneous bone marrow grafting of delayed union and nonunion in cancer patients. Clinical Orthopaedics & Related Research 256:280-285, 1990.
56. Heckman JD, Boyan BD, Aufdemorte TB, and Abbott JT.: The use of bone morphogenetic protein in the treatment of non-union in a canine model. Journal of Bone & Joint Surgery American 73:750-64, 1991.
57. Holmes RE, Bucholz RW, and Mooney V.: Porous hydroxyapatite as a bone graft substitute in diaphyseal defects: a histometric study. Journal of Orthopaedic Research 5:114-21, 1987.
58. Holocek, J.; Lennon, D.L., Haynesworth, S.E.; Marshak, D.R.; and Caplan, A.I: Unpublished data.
59. Huang S, and Terstappen LW.: Formation of haematopoietic microenvironment and haematopoietic stem cells from single human bone marrow stem cells [retraction of Huang S, Terstappen LW. In: Nature 1992 Dec 24-31;360(6406):745-9]. Nature 368:1994.
60. Huang S, and Terstappen LW.: Formation of haematopoietic microenvironment and haematopoietic stem cells from single human bone marrow stem cells [see comments] [retracted by Huang S, Terstappen LW. In: Nature 1994 Apr 14;368(6472):664]. Nature 360:745-9, 1992.
61. Hunt, T.R.; Schwappach, J.R.; and Anderson, H.C.: Healing of a segmental defect in the rat femur with use of an extract from a cultured human osteosarcoma cell-line (Saos-2). J. Bone Joint Surg. 78(1):41-48, 1996.
62. Ishida H, Bellows CG, Aubin JE, and Heersche JN.: Characterization of the 1,25-(OH)2D3-induced inhibition of bone nodule formation in long-term cultures of fetal rat calvaria cells. Endocrinology 132:61-6, 1993.
63. Jaiswal, N. and Bruder, S.P. Trans. O.R.S.: 524, 1997.
64. Jaiswal, N.; Haynesworth, S.E.; Caplan, A.I.; and Bruder, S.P.: Osteogenic differentiation of purified, culture-expanded human mesenchymal stem cells in vitro, (1997) J. Cell Biochem. 64(2):295-312.
65. Johnson KA, Howlett CR, Bellenger CR, and Armati GP.: Osteogenesis by canine and rabbit bone marrow in diffusion chambers. Calcified Tissue International 42:113-8, 1988.
66. Johnstone, B.; Yoo, J.U.; Barry, F.P.: In vitro chondrogenesis of bone marrow-derived mesenchymal cells. Trans. Ortho. Res. Soc. 21: 65, 1996.
67. Joyce ME, Roberts AB, Sporn MB, and Bolander ME.: Transforming growth factor-beta and the initiation of chondrogenesis and osteogenesis in the rat femur. Journal of Cell Biology 110:2195-207, 1990.
68. Kadiyala, S., Jaiswal, N., and Bruder, S.P. (1997) Tissue. Engin. 3, (in press).
69. Kadiyala, S., Kraus, K.H., and Bruder, S.P. (1996) Trans. Tissue. Engin. Soc. 1, 20.
70. Kadiyala, S., Young, R.G., Thiede, M.A., and Bruder, S.P. (1997) Cell Transplant. 6, (in press).
71. Kahanovitz N, and Arnoczky SP.: The efficacy of direct current electrical stimulation to enhance canine spinal fusions. Clinical Orthopaedics & Related Research 251:295-299, 1990.
72. Kahn, A.; Gibbons, R.; Perkins, S.; and Gazit, D.: Age-related bone loss: A hypothesis and initial assessment in mice. Clin. Orthop. Rel. Res. 313:69-75, 1995.
73. Lane JM, and Sandhu HS.: Current approaches to experimental bone grafting. Orthopedic Clinics of North America 18:213-25, 1987.
74. Lane, J. M.; Yasko, A.; Tomin, E.; Bostrom, M.; Rosen, V.; and Wozney, J.: Orthopaedic application of BMP-2 in fracture healing. In First International Conference on Bone Morphogenic Proteins, Baltimore, MD, June 8-11 (abstract), 1994.
75. Laurie, S.W.S.; Kaban, L.B.; Mulliken, J.B.; and Murray, J.E.: Donor-site morbidity after harvesting rib and iliac bone. Plast. Reconstr. Surg. 73(6):933-938, 1984.
76. Leads from the MMWR. Transmission of HIV through bone transplantation: Case report and publich health recommendations. JAMA. 260:2487-2488, 1988.
77. LeBoy, P.S.; Beresford, J.; Devlin, C.; and Owen, M.: Dexamethasone induction of osteoblast mRNAs in rat marrow stromal cell cultures. J. Cell Physiol. 146:370-378, 1991
78. Lee, S.C., Shea, M., Battle, M.A., Kozitza, K., Ron, E., Turek, T., Schaub, R.G., and Hayes, W.C. (1994) J. Biomed. Mater. Res. 28, 1149-1156.
79. Lennon, D.P.; Haynesworth, S.E.; Young, R.G.; Dennis, J.E.; and Caplan, A.I.: A chemically defined medium supports in vitro proliferation and maintains the osteochondral potential of rat marrow-derived mesenchymal stem cells. Exp. Cell Res. 219:211-222, 1995.
80. Lennon, D.P.; Haynesworth, S.E.; Bruder, S.P.; Jaiswal, N.; and Caplan, A.I.: Human and animal mesenchymal progenitor cells from bone marrow: Identification of serum for optimal selection and proliferation. In Vitro Cell. Dev. Biol., 32(10):602-611, 1996.
81. Lian JB, and Stein GS.: Concepts of osteoblast growth and differentiation: basis for modulation of bone cell development and tissue formation. [Review]. Critical Reviews in Oral Biology & Medicine 3:269-305, 1992.
82. Liang, C.T.; Barnes, J.; Seedor, J.G; Quartuccio, H.A.; Bolander, M.; Jeffrey, J.J.; and Rodan, G.A.: Impaired bone activity in aged rats: Alterations at the cellular and molecular levels. Bone. 13:435-441, 1992.
83. Liebergall, M.; Young, R. G.; Ozawa, N.; Reese, J.; Davy, D. T.; Goldberg, V. M.; and Caplan, A. I.: The effects of cellular manipulation and TGF-§ in a composite bone graft. In: Bone Formation and Repair. Brighton, C., Friedlander, G., and Lane, J. (eds), American Academy of Orthopaedic Surgeons, Rosemont, Il, 367-378, 1994.
84. Lindholm TS, Ragni P, and Lindholm TC.: Response of bone marrow stroma cells to demineralized cortical bone matrix in experimental spinal fusion in rabbits. Clinical Orthopaedics & Related Research 150:296-302, 1988.
85. Lindholm TS, and Urist MR.: A quantitative analysis of new bone formation by induction in compositive grafts of bone marrow and bone matrix. Clinical Orthopaedics & Related Research 150:288-300, 1980.
86. Lovell TP, Dawson EG, Nilsson OS, and Urist MR.: Augmentation of spinal fusion with bone morphogenetic protein in dogs. Clinical Orthopaedics & Related Research 243:266-274, 1989.
87. Majumdar, M.K.; Haynesworth, S.E.; Thiede, M.A.; Marshak, D.R.; Caplan, A.I.; and Gerson, S.L.: Culture-expanded human mesenchymal stem cells (MSCs) express cytokines and support hematopoiesis in vitro. Blood 86(10):494a (1995).
88. Malaval, L.; Modrowski, D.; Ashwani, G.; and Aubin, J.E.: Cellular expression of bone-related proteins during in vitro osteogenesis in rat bone marrow stromal cell cultures. J. Cell Physiol. 158:555-572, 1994.
89. Maniatopolous, C.; Sodek, J.; and Melcher, A.H.: Bone formation in vitro by stromal cells obtained from bone marrow of young adult rats. Cell Tiss. Res. 254:317-330, 1988.
90. McDavid PT, Boone M2, Kafrawy AH, and Mitchell DF.: Effect of autogenous marrow and calcitonin on reactions to a ceramic. Journal of Dental Research,58:147883, 1979.
91. Moore DC, Chapman MW, and Manske D.: The evaluation of a biphasic calcium phosphate ceramic for use in grafting long-bone diaphyseal defects. Journal of Orthopaedic Research 5:356-65, 1987.
92. Mosca, J.D., Majumdar, M.K., Hardy, W.B., Pittenger, M.F., and Thiede, M.A. (1997) Blood 88(10), 186a.
93. Mulliken JB, Kaban LB, and Glowacki J.: Induced osteogenesis-the biological principle and clinical applications. Journal of Surgical Research 37:487-96, 1984.
94. Muschler GF, Huber B, Ullman T, et al.: Evaluation of bone-grafting materials in a new canine segmental spinal fusion model. Journal of Orthopaedic Research 11:514-24, 1993.
95. Muschler GF, Hyodo A, Manning T, Kambic H, and Easley K.: Evaluation of human bone morphogenetic protein 2 in a canine spinal fusion model. Clinical Orthopaedics & Related Research 308:229-240, 1994.
96. Muschler GF, Lane JM, and Dawson EG. The biology of spinal fusion.New York: Springer-Verlag, 1 990:9-21. (Cotler JM, Cotler HB, ed. Science and techniques
97. Muschler GF, Negami S, Kambic H, and Easley K.: The evaluation of collagen and ceramic composites as bone graft materials in a canine posterior segmental spinal fusion model. Submitted to Clinical Orthopaedics & Related Research.
98. Muscolo DL, Caletti E, Schajowicz F, Araujo ES, and Makino A.: Tissue-typing in human massive allografts of frozen bone, Journal of Bone & Joint Surgery American 69:583-95, 1987.
99. Muthukumaran N, and Reddi AH.: Bone matrix-induced local bone induction. [Review]. Clinical Orthopaedics & Related Research 200:159-64, 1985.
100. Nade S, Armstrong L, McCartney E, and Baggaley B.: Osteogenesis after bone and bone marrow transplantation. The ability of ceramic materials @o sustain osteogenesis from transplanted bone marrow cells: preliminary studies. Clinical Orthopaedics & Related Research 181:255-63, 1983.
101. Nade S, and Burwell RG.: Decalcified bone as a substrate for osteogenesis. An appraisal of the interrelation of bone and marrow in combined grafts. Journal of Bone & Joint Surgery British 59:189-96, 1977.
102. Nakahara H, Dennis JE, Bruder SP, Haynesworth SE, Lennon DP, and Caplan Al.: in vitro differentiation of bone and hypertrophic cartilage from periosteal-derived cells. Experimental Cell Research 195:492-503, 1991.
103. Niedzwiedzki, T.; Dabrowski, Z.; Miszta, H.; and Pawlikowski, M.: Bone healing after bone marrow stromal cell transplantation to the bone defect. Biomaterials 14:115-121, 1993.
104. Nilsson OS, Urist MR, Dawson EG, Schmalzried TP, and Finerman GA.: Bone repair induced by bone morphogenetic protein in ulnar defects in dogs. Journal of Bone & Joint Surgery British 68:635-42, 1986.
105. Ohgushi, H.; Goldberg, V.M.; and Caplan, A. I.: Repair of bone defects with marrow cells and porous ceramic: Experiments in rats. Acta. Orthop. Scand. 60:334-339, 1989.
106. Ohgushi H, Goldberg VM, and Caplan Al.: Heterotopic osteogenesis in porous ceramics induced by marrow cells. Journal of Orthopaedic Research 7:568-78, 1989.
107. Oikarinen J.: Experimental spinal fusion with decalcified bone matrix and deep-frozen allogeneic bone in rabbits. Clinical Orthopaedics & Related Research 162:210-218, 1982.
108. Ou Y, Piedmonte MR, and Medendrop SV.: Latent variable models for clustered ordinal data. Submitted to Biometrics.
109. Owen, M.; Lineage of osteogenic cells and their relationship to the stromal system. In Bone and Mineral/3. W.A. Peck, ed. Elsevier, Amsterdam, 1-25,1985.
110. Owen, M.: Marrow stromal stem cells. J. Cell Sci. Suppl. 10:63-76, 1988.
111. Owen M, and Friedenstein AJ.: Stromal stem cells: marrow-derived osteogenic precursors. [Review]. Ciba Foundation Symposium 136:42-60, 1988.
112. Paik D.-. Repeated measurement analysis for non-normal data in small samples. Commun Statist Simulation 17:1155-1171, 1988.
113. Paley, D.; Young, M.C.; Wiley, A.M.; Fornasier, V.L.; and Jackson, R.W.: Percutaneous bone marrow grafting of fractures and bone defects. Clin. Orthop. Rel. Res. 208:300-311, 1986.
114. Pelker RR, McKay JJ, Troiano N, Panjabi MM, and Friedlaender GE.: Allograft incorporation- a biomechanical evaluation in a rat model. Journal of Orthopaedic Research 7:585-9, 1989.
115. Pereira, R. F.; Halford, K. W.; O'Hara, M. D.; Leeper, D. B.; Sokolov, B. P.; Pollard, M. D.; Bagasra, O.; and Prockop, D. J.: Culture adherent cells from marrow can serve as long-lasting precursor cells for bone, cartilage, and lung in irradiated mice. Proc. Natl. Acad. Sci. USA. 92:4857-4861, 1988.
116. Pittenger, M.F., Mackay, A.M., and Beck, S.C. (1996) Mol. Biol. Cell. 7, 582a.
117. Preiffer CA.-. Development of bone from transplanted marrow in mice. Anat Rec 102:225, 1948.
118. Quarto, R.; Thomas, D.; and Liang, T.: Bone progenitor cell deficits and the age-associated decline in bone repair capacity. Calcif. Tissue Int. 56:123-129, 1995.
119. Ragni P, Lindholm TS, and Lindholm TC.: Vertebral fusion dynamics in the thoracic and lumbar spine induced by allogenic demineralized bone matrix combined with autogenous bone marrow. An experimental study in rabbits. Italian Journal of Orthopaedics & Traumatology 13:241-51, 1987.
120. Rejda BV, Peelen JG, and de GK.: Tri-calcium phosphate as a bone substitute. Journal of Bioengineering 1:93-7, 1977.
121. Saito, T.; Dennis, J.E.; Lennon, D.P.; Young, R.G.; and Caplan, A.I.: Myogenic expression of mesenchymal stem cells within myotubes of mdx mice in vitro and in vivo. Tissue. Engin. 1(4):327-343, 1995.
122. Salama R, and Weissman SL.: The clinical use of combined xenografts of bone and autologous red marrow. A preliminary report. Journal of Bone & Joint Surgery British 60:111-5, 1978.
123. Schuurman, H.-J., Hougen, H.P., and van Loveren, H. (1992) ILAR Journal. 34(1-2), 3-12.
124. Simmons DJ, Ellsasser JC, Cummins H, and Lesker P.: The bone inductive potential of a composite bone allograft-marrow autograft in rabbits. Clinical Orthopaedics & Related Research 97:237-47, 1973.
125. Stabler CL, Eismont FJ, Brown MD, Green BA, and Malinin Ti.: Failure of posterior cervical fusions using cadaveric bone graft in children. Journal of Bone & Joint Surgery American 67:371-5, 1985.
126. Stevenson, S.; Cunningham, N.; Toth, J.; Davy, D.; and Reddi, A. H.: The effect of osteogenin (a bone morphogenic protein) on the formation of bone in orthotopic segmental defects in rats. J. Bone Joint Surg. 76(11):1676-1687. 1994.
127. Stevenson S, Hohn RB, and Templeton JW.: Effects of tissue antigen matching on the healing of fresh cancellous bone allografts in dogs. American Journal of Veterinary Research 44:201-6, 1983.
128. Tabuchi, C.; Simmon, D.J.; Fausto, A.; Russell, J.; Binderman, I.; and Avioli, L.: Bone deficit in ovariectomized rats. J. Clin. Invest. 78:637-642, 1986.
129. Takagi, K.; and Urist, M. R.: The role of bone marrow in bone morphogenic protein-induced repair of femoral massive diaphyseal defects. Clin. Orthop. Rel. Res. 171:224-231, 1982.
130. Thomas ED, and Storb R.: Technique for human marrow grafting. Blood 36:507-15, 1970.
131. Thomas 1, Kirkaidy WW, Singh S, and Paine KW.: Experimental spinal fusion in guinea pigs and dogs: the effect of immobilization. Clinical Orthopaedics & Related Research 112:363-375, 1975.
132. Tiedeman, J.J.; Connolly, J.F.; Strates, B.S.; and Lippiello, L.: Treatment of nonunion by percutaneous injection of bone marrow and demineralized bone matrix. Clin. Orthop. Rel. Res. 268:294-302, 1991.
133. Tiedeman, J.J.; Huurman, W.W.; Connolly, J.F.; and Strates, B.S.: Healing of a large nonossifying fibroma after grafting with bone matrix and marrow. Clin. Orthop. Rel. Res. 265:302-305, 1991.
134. Tomford WW, Starkweather RJ, and Goldman MH.: A study of the clinical incidence of infection in the use of banked allograft bone. Journal of Bone & Joint Surgery American 63:244-8, 1981.
135. Tsuji, T.; Hughhes, F.J.; McCulloch, C.A.; and Melchher, A.H.: Effect of donor age on osteogenic cells of rat bone marrow in vitro. Mech. Ageing Dev. 51:121-132, 1990.
136. Tuli SM, and Singh AD.: The osteoninductive property of decalcified bone matrix. An experimental study,. Journal of Bone & Joint Surgery British 60:116-23, 1978.
137. Turksen K, and Aubin JE.: Positive and negative immunoselection for enrichment of two classes of osteoprogenitor cells. Journal of Cell Biology 114:37384, 1991.
138. Urist, M.R.: Bone: Formation by autoinduction. Science 150:893-899, 1965.
139. Urist MR, and Dawson E.: Intertransverse process fusion with the aid of chemosterilized autolyzed antigen-extracted allogeneic (AAA) bone. Clinical Orthopaedics & Related Research 154:97-113, 1981.
140. Urist MR, DeLange RJ, and Finerman GA.: Bone cell differentiation and growth factors. Science 220:680-6, 1983.
141. Villanueva JE, and Nimni ME.: Promotion of calvarial cell osteogenesis by endothelial cells. Journal of Bone & Mineral Research 5:733-9, 1990.
142. Wakitani, S.; Gotto, T.; Pineda, S. J.; Young, R. G.; Mansour, J. M.; Caplan, A. I.; and Goldberg, V. M.: Mesenchymal cell-based repair of large, full-thickness defects of articular cartilage J. Bone Joint Surg. 76A:579-592, 1994.
143. Wakitani, S.; Saito, T.; and Caplan, A.I.: Myogenic cells derived from rat bone marrow mesenchymal stem cells exposed to 5-azacytidine. Muscle & Nerve 18:1417-1426, 1995.
144. Werntz, J.R.; Lane, J.M.; Burstein, A.H.; Justin, R.; Klein, R.; and Tomin, E.: Qualitative and quantitative analysis of orthotopic bone regeneration by marrow. J. Orthop. Res. 14:85-93, 1996.
145. Wolff, D.; Goldberg, V. M.; and Stevenson, S.: Histomorphometric analysis of the repair of a segmental diaphyseal defect with ceramic and titanium fibermetal implants: Effects of bone marrow. J. Orthop. Res. 12:439-446, 1994.
146. Wozney, J.M.; Rosen, V.; Celeste, A.J.; Mitsock, L.M.; Whitters, M.J.; Kriz, R.W.; Hewick, R.M.; and Wang, E.A.: Novel regulators of bone formation: Molecular clones and activities. Science. 242:1528-1534, 1988.
147. Yasko, A. W.; Lane, J. M.; Fellinger, E. J.; Rosen, V.; Wozney, J. M.; and Wang, E. A.: The healing of segmental bone defects, induced by recombinant human bone morphogenic protein (rhBMP-2). J. Bone Joint Surg. 74(5):659-671, 1992.
148. Young, R.G., Butler, D.L., Weber, W., Gordon, S.L., and Fink, D.J. (1997) Trans. Ortho. Res. Soc. 22, 249.
149. Younger, E.M.; and Chapman, M.W.: Morbidity at bone graft donor sites. J. Orthop. Trauma. 3:192-195, 1989.
150. Zeger SL, Liang KY, and Albert PS.: Models for longitudinal data: a generalized estimating equation approach [published erratum appears in Biometrics 1989 Mar;45(1):347]. Biometrics 44:1049-60, 1988.
151. Zerwekh JE, Kourosh S, Scheinberg R, et al.: Fibrillar collagen-biphasic calcium phosphate composite as a bone graft substitute for spinal fusion. Journal of Orthopaedic Research 10:562-72, 1992.

## Claims

1. A pharmaceutical composition comprising a porous ceramic material and isolated mesenchymal stem cells for use in the treatment of a bone defect wherein the treatment comprises the administration of the composition into the bone defect.

2. The composition of claim 1, wherein the bone defect is a segmental bone defect.

3. The composition of claim 1 or 2, wherein the ceramic material is selected from the group consisting of hydroxyapatite, β-tricalcium phosphate, and combinations thereof.

4. The composition of any one of claims 1 to 3, wherein the ceramic material is in particulate form.

5. The composition of any one of claims 1 to 3, wherein the ceramic is a structurally stable, three-dimensional implant.

6. The composition of claim 5, wherein the structurally stable, three-dimensional implant is a cube, cylinder, block or in the shape of an anatomical form.

7. The composition of any one of the preceding claims, further comprising at least one bioactive factor that induces or accelerates the differentiation of mesenchymal stem cells into the osteogenic lineage.

8. The composition of claim 7, wherein the bioactive factor is a synthetic glucocorticoid or a bone morphogenic protein.

9. The composition of claim 8, wherein the synthetic glucocorticoid is dexamethasone.

10. The composition of claim 8, wherein the bone morphogenic protein is selected from the group consisting of BMP-2, BMP-3, BMP-4, BMP-6, and BMP-7.

11. The composition of any one of claims 1 to 10, wherein the treatment of the bone defect results in healing of the bone defect.

12. Use of a pharmaceutical composition of any one of claims 1 to 11 for the preparation of a medicament for the treatment of a bone defect wherein the treatment comprises the administration of the composition into the bone defect.

13. Use of a porous ceramic material and isolated mesenchymal stem cells for the preparation of a pharmaceutical composition for the treatment of a bone defect wherein the treatment comprises the administration of the composition into the bone defect.

14. The use of claim 13, wherein the bone defect is a segmental bone defect.

15. The use of claim 13 or 14, wherein the ceramic material is selected from the group consisting of hydroxyapatite, β-tricalcium phosphate, and combinations thereof.
